# EUROPÄISCHE PATENTSCHRIFT

(11) **EP 0 890 343 B1**
(45) Veröffentlichungstag und Bekanntmachung des Hinweises auf die Patenterteilung: **01.10.2003**
(21) Anmeldenummer: 98112002.5
(22) Anmeldetag: 29.06.1998
(51) Int. Cl.: A61C 1/00, A61B 17/16

(54) **Ärztliche Behandlungsvorrichtung**
Medical treatment device
Dispositif de traitement médical

(30) Priorität: 08.07.1997 DE 19729177
(43) Veröffentlichungstag der Anmeldung: 13.01.1999
(73) Patentinhaber: Kaltenbach & Voigt GmbH & Co. KG, 88400 Biberach/Riss (DE)
(72) Erfinder: Eibofner, Eugen, 88400 Bieberach (DE); Strohmaier, Ernst, 88427 Bad Schussenried (DE); Ferraro, Michele, 4565 Recherswil (CH)
(74) Vertreter: Schmidt-Evers, Jürgen, Dipl.-Ing.

(56) Entgegenhaltungen:
- DE-A- 3 144 855
- JP-A- 60 090 560
- US-A- 5 538 423

## Beschreibung

Die vorliegende Erfindung betrifft eine ärztliche Behandlungsvorrichtung. Insbesondere betrifft die vorliegende Erfindung eine zahnärztliche Behandlungsvorrichtung, welche im Bereich der Mikrochirurgie, HNO-Chirurgie, der plastischen Chirurgie und der Kieferchirurgie sowie der Implantologie eingesetzt werden kann.

Bekannterweise besteht eine derartige ärztliche Behandlungsvorrichtung aus einem elektronischen Steuergerät, an das mit Hilfe einer biegsamen bzw. flexiblen Versorgungsleitung ein Behandlungsinstrument in Form eines Handstücks, z.B. in Form eines zahnärztlichen Handstücks oder eines zahnärztlichen Winkelstücks, anschließbar ist, welches an seinem vorderen Ende ein Behandlungswerkzeug trägt, das vorzugsweise austauschbar ist. Der Antrieb des Behandlungsinstruments bzw. des Behandlungswerkzeugs erfolgt mit Hilfe eines Antriebsmotors, der an die Versorgungsleitung angeschlossen und mit dem Behandlungsinstrument koppelbar ist. Bei bestimmten Behandlungsinstrumenten bzw. Behandlungswerkzeugen. wie z.B. Bohrern, ist die Zuführung eines Kühlmittels zur Behandlungsstelle erforderlich. Zu diesem Zweck ist eine Kühlmittelpumpe vorgesehen, die vorzugsweise am Steuergerät angebaut oder in dieses integriert sein kann. Über eine Kühlmittelleitung wird der Behandlungsstelle eine Kühlflüssigkeit, wie z.B. eine NaCl-Lösung, zugeführt, wobei die Kühlmittelleitung zusätzlich zu der Versorgungsleitung vorgesehen oder zumindest teilweise in die Versorgungsleitung bzw. das Handstück integriert sein kann.

Das Steuergerät steuert zum einen den Betrieb der Kühlmittelpumpe, d.h. die zugeführte Kühlmittelmenge, und zum anderen den Betrieb des Behandlungsinstruments bzw. Behandlungswerkzeugs über den Antriebsmotor. Das Steuergerät steuert demnach sowohl die Drehzahl als auch das Drehmoment des Antriebsmotors, um ein bestimmtes Behandlungsinstrument bzw. Behandlungswerkzeug mit den jeweils geeigneten Betriebsparametern zu betreiben.

Von der Firma W&H Dentalwerk Bürmoos GmbH ist unter der Bezeichnung Elcomed 100/200 ein zahnärztliches Chirurgiegerät bekannt, bei dem das Steuergerät drei Programmtasten aufweist. Jeder Programmtaste ist herstellerseitig ein bestimmtes Behandlungsprogramm zugeordnet. Dabei umfaßt jedes Behandlungsprogramm eine voreingestellte Drehzahl, ein voreingestelltes Drehmoment, ein voreingestelltes Übersetzungsverhältnis und eine voreingestellte Kühlmittelpumpenfördermenge. Durch Betätigen einer dieser drei Programmtasten werden die dem ausgewählten Betriebs- bzw. Behandlungsprogramm zugeordneten Betriebsparameter auf einem Display angezeigt, welches in der Frontseite des Steuergeräts integriert ist. Wird zwischen den einzelnen Betriebsprogrammen umgeschaltet, werden die zuletzt eingestellten Werte gespeichert, d.h. eine manuelle Einstellung oder Verstellung einer Drehzahl usw. geht nicht verloren und die drei Programmtasten können mit beliebigen benutzerspezifischen Betriebsprogrammen belegt werden. Beim Abspeichern eines benutzerspezifischen Betriebsprogramms geht jedoch das der entsprechenden Programmtaste zugeordnete herstellerseitige Betriebsprogramm verloren. Um die vom Hersteller vorgegebenen Betriebsprogramme wieder abrufen zu können, muß eine der Programmtasten beim Einschalten des Steuergeräts für eine bestimmte Zeitdauer gedrückt werden. Anschließend wird mit Hilfe eines Piepstons angezeigt, daß nun wieder die werkseitig vorgegebenen Betriebsprogramme der drei Programmtasten vorhanden sind, wobei jedoch ggfs. zuvor abgespeicherte benutzerspezifische Betriebsprogramme mit veränderten Drehzahl- oder Drehmomentwerten verloren gehen.

Bei dem zuvor beschriebenen Chirurgiegerät sind jedoch folgende Umstände problematisch. Zum einen stehen dem Benutzer lediglich begrenzte Betriebsprogramme zur Verfügung. Da lediglich drei Programmtasten an dem Steuergerät vorgesehen sind, kann der Benutzer nur zwischen drei vorgegebenen Betriebsprogrammen auswählen, was jedoch hinsichtlich der Vielzahl an Kombinationen von Behandlungsinstrumenten und Behandlungswerkzeugen mit unterschiedlichen Drehmomenten und Drehzahlen für die einzelnen Anwendungen nicht bedienfreundlich ist. Zum anderen wird beim Abspeichern eines benutzerspezifisch erstellten Behandlungsprogramms unweigerlich ein unter derselben Programmtaste zuvor abgelegtes Behandlungsprogramm überschrieben und geht somit verloren. Dies ist insbesondere ärgerlich, wenn es sich bei dem zuvor unter derselben Programmtaste abgelegten Behandlungsprogramm um ein herstellerseitig vorgegebenes Behandlungsprogramm handelt, da hersteller- bzw. werkseitig in der Regel sinnvolle Kombinationen für Drehzahl-, Drehmoment- und Übersetzungsverhältniswerte vorgegeben werden. Die herstellerseitig vorgegebenen Betriebsprogramme können jedoch nur durch einen komplizierten und nicht bedienfreundlichen Resetvorgang wiedergewonnen werden, der ein Aus- und erneutes Einschalten des Chirurgiegeräts voraussetzt. Dabei gehen jedoch wiederum die zuvor abgelegten benutzerspezifischen Betriebsprogramme verloren. Bei dem bekannten, zuvor beschriebenen Chirurgiegerät kann demnach ein Benutzer nicht gleichzeitig zwischen beispielsweise drei herstellerseitig vorgegebenen Behandlungsprogrammen und drei benutzerspezifischen Behandlungsprogrammen auswählen, die zuvor von dem Benutzer selbst erstellt und abgespeichert worden sind. Insgesamt ist bei dem bekannten Chirurgiegerät die Auswahlmöglichkeit zwischen einzelnen Behandlungsprogrammen und damit die Bedienfreundlichkeit stark eingeschränkt.

Aus der US 5,538,423 ist ebenfalls ein zahnärztliches Chirurgiegerät bekannt, bei dem verschiedene Betriebsparameter-Kombinationen als Programme gespeichert werden können. Bei Inbetriebnahme des Geräts sind in dem Speicher für die Betriebsprogramme Basis- oder Grundwerte gespeichert, die bei einer Programmierung und Speicherung von benutzerspezifischen Programmen überschrieben werden. Auch hier ist es somit nicht und zumindest nur unter großem Aufwand möglich, auf die herstellerseitig vorgegebenen Einstellungen zurückzugreifen. Schließlich ist auch aus der DE 31 44 855 eine Steuereinrichtung für ein zahnärztliches Gerät bekannt, bei der Betriebsprogramme durch eine Eingabeleitung veränderbar sind.

Der vorliegenden Erfindung liegt die Aufgabe zugrunde, eine ärztliche Behandlungsvorrichtung der zuvor beschriebenen Art derart auszugestalten, daß ein Benutzer hinsichtlich der Auswahlmöglichkeit zwischen werk- bzw. herstellerseitig vorgegebenen Betriebsprogrammen einerseits und benutzerspezifisch erstellten Betriebsprogrammen andererseits nicht mehr beschränkt ist, so daß die Bedienfreundlichkeit der ärztlichen Behandlungsvorrichtung deutlich erhöht und der Vielzahl von unterschiedlichen Kombinationen von Behandlungsinstrumenten und Behandlungswerkzeugen mit unterschiedlichen Drehzahl-, Drehmoment-, Übersetzungsverhältnis- und Kühlmittelwerten usw. Rechnung getragen wird.

Diese Aufgabe wird erfindungsgemäß durch eine ärztliche Behandlungsvorrichtung gemäß Anspruch 1 gelöst.

Die Unteransprüche beschreiben allgemein vorteilhafte Ausgestaltungen und bevorzugte Ausführungsbeispiele der vorliegenden Erfindung.

Die erfindungsgemäße ärztliche Behandlungsvorrichtung umfaßt mindestens zwei Programmebenen. In einer ersten Programmebene kann der Benutzer zwischen einer Vielzahl von hersteller- bzw. werkseitig vorgegebenen Behandlungs- bzw. Betriebsprogrammen auswählen. In einer zweiten Programmebene kann der Benutzer zwischen einer Vielzahl von benutzerspezifisch erstellten Betriebsprogrammen auswählen. Nach Auswahl eines der Betriebsprogramme steuert das Steuergerät das Behandlungswerkzeug bzw. Behandlungsinstrument abhängig von den dem ausgewählten Betriebsprogramm entsprechenden Betriebsparameterinformationen. Der Benutzer kann beliebig zwischen den benutzerspezifisch abgespeicherten Betriebsprogrammen und den herstellerseitig vorgegebenen Betriebsprogrammen wählen und umschalten. Jeder Auswahltaste zur Auswahl der beiden zuvor beschriebenen Programmebenen kann eine im Prinzip beliebige Anzahl von herstellerseitig vorgegebenen bzw. benutzerspezifisch erstellten Betriebsprogrammen zugeordnet sein, wobei insbesondere jede Taste mit 100 unterschiedlichen Betriebsprogrammen belegt sein kann. Nach Auswahl einer der beiden Programmebenen kann innerhalb der Programmebene aus den entsprechenden Betriebsprogrammen ausgewählt werden.

Die jedem Betriebsprogramm zugeordneten Betriebsparameterinformationen enthalten Informationen über ein auszuwählendes Behandlungsinstrument, z.B. ein dentales Handstück oder ein dentales Winkelstück oder Hand- und Winkelstückparameter wie z.B. Drehzal, Untersetzungsverhältnis, Wirkungsgrad usw., und ein auszuwählendes Behandlungswerkzeug, wie z.B. einen Bohrer. Für die dadurch festgelegte Behandlungsinstrument-Behandlungswerkzeug-Kombination ist zudem durch die Betriebsparameterinformationen ein entsprechender Betriebsdrehzahlbereich sowie das gewünschte Drehmoment vorgegeben. Zudem können die Betriebsparameterinformationen Informationen über die zuzuführende Kühlmittelmenge enthalten.

Das Steuergerät besitzt eine Datenbank, welche Informationen über bestimmte verfügbare Behandlungsinstrumente und Behandlungswerkzeuge umfaßt. Zudem können in der Datenbank Informationen über beispielsweise vorgegebene Kühlmittelmengen gespeichert sein. Ein Benutzer kann mit Hilfe einer zusätzlichen Auswahltaste einen Einstellmodus auswählen, in dem der Benutzer durch Auswahl bestimmter Informationen dieser Datenbank ein eigenes benutzerspezifisches Betriebsprogramm erstellt. Neben den zuvor beschriebenen beiden Programmebenen ist somit eine dritte Betriebsebene vorgesehen. Nach der manuellen Einstellung der gewünschten Betriebsparameter kann der Benutzer dieses von ihm erstellte Betriebsprogramm als ein benutzerspezifisches Behandlungs- bzw. Betriebsprogramm abspeichern.

In der Frontseite des Steuergeräts ist eine Anzeigeneinheit, z.B. eine Flüssigkristallanzeige, integriert, die die geltenden ausgewählten bzw. manuell eingestellten Betriebsparameterinformationen anzeigt. Zudem kann das Steuergerät mit einer Hilfefunktion ausgestattet sein, so daß zu den einzelnen Funktionen des Steuergeräts Hilfeinformationen bzw. Hilfetexte auf der Anzeigeneinheit dargestellt werden können. Des weiteren kann eine Fehlerfunktion in dem Steuergerät integriert sein, mit deren Hilfe über die Anzeigeneinheit Fehlermeldungen bei Auftreten eines Fehlerfalls in dem Steuergerät, wie z.B. eine Überhitzung des Antriebsmotors oder des Netzteils oder eine vollständige Belegung der beiden Programmebenen, angezeigt werden kann.

Gemäß einer bevorzugten Ausführungsform kann die ärztliche Behandlungsvorrichtung eine zweite Kühlmittelpumpe aufweisen, die zusammen mit einem zweiten an das Steuergerät anschließbaren Antriebsmotor betrieben werden kann, so daß zwei Antriebsmotoren mit entsprechend daran angekoppelten Behandlungsinstrumenten und Behandlungswerkzeugen unabhängig voneinander betrieben werden können. Vorzugsweise besitzen die beiden Antriebsmotoren unterschiedliche Drehzahl- bzw. Drehmomentbereiche.

Des weiteren kann die ärztliche Behandlungsvorrichtung eine Pneumatik-Antriebseinrichtung aufweisen, um bestimmte Behandlungswerkzeuge pneumatisch über einen Luftdruckanschluß betreiben zu können. Der Antriebsluftdruck kann dabei beliebig reguliert und eingestellt werden.

Über eine serielle oder parallele Schnittstelle kann an das Steuergerät ein Drucker angeschlossen werden, über den die jeweils geltenden Betriebsparameterinformationen sowie weitere Betriebsinformationen, wie z.B. die Uhrzeit oder das Datum, ausgedruckt werden können, so daß die Behandlung auch nachträglich beurteilt werden kann. Ebenso kann eine Schnittstelle zum Anschluß des Steuergeräts an einen Computer vorgesehen sein, um Daten an den Computer übertragen zu können und am Bildschirm des Computers eine vergrößerte Displayanzeige darstellen bzw. mit Hilfe des Computers Daten aufzeichnen zu können.

Die Funktionen des Steuergeräts können zum einen über entsprechend an dem Steuergerät vorgesehene Auswahltasten und zum anderen über einen Fußschalter ausgewählt werden. Der Fußschalter ist vorzugsweise als kabelloser Fußschalter ausgestaltet und überträgt die entsprechenden Informationssignale beispielsweise über eine Infrarot- oder Funk-Übertragungsstrecke an eine entsprechend vorgesehene Empfangseinheit des Steuergeräts. Mit Hilfe des Fußschalters kann sowohl der Antriebsmotor direkt angesteuert als auch ein bestimmtes herstellerseitig vorgegebenes bzw. benutzerspezifisch abgespeichertes Betriebsprogrammen ausgewählt werden.

Die erfindungsgemäße ärztliche Behandlungsvorrichtung weist gegenüber dem eingangs beschriebenen Stand der Technik den Vorteil auf, daß der Benutzer stets zwischen herstellerseitig vorgegebenen Betriebsprogrammen und benutzerspezifisch erstellten Betriebsprogrammen auswählen kann. Das Auswählen dieser beiden Programmebenen erfolgt vorzugsweise über entsprechend vorgesehene Auswahltasten, wobei nach Auswahl einer dieser beiden Programmebenen zwischen den der ausgewählten Programmebene zugeordneten Betriebsprogrammen gewählt werden kann. Auf diese Weise kann jede der beiden Programmebenen, d.h. jede der den beiden Programmebenen zugeordneten Auswahltasten, mit einer beliebigen Anzahl von Betriebsprogrammen belegt werden, wobei die zur Verfügung stehende Gesamtanzahl an Betriebsprogrammen gegenüber dem bekannten Stand der Technik deutlich erhöht ist.

Des weiteren ist ein Einstellmodus vorgesehen, in dem der Benutzer die Betriebsparameter beliebig einstellen und somit ein neues Betriebsprogramm generieren kann.

Insgesamt konnte mit Hilfe der erfindungsgemäßen ärztlichen Behandlungsvorrichtung die Bedienfreundlichkeit gegenüber dem bekannten Stand der Technik deutlich erhöht werden.

Die Erfindung wird nachfolgend anhand bevorzugter Ausführungsbeispiele unter Bezugnahme auf die beigefügte Zeichnung näher beschrieben.
- Fig. 1: zeigt ein bevorzugte Ausführungsbeispiel einer erfindungsgemäßen ärztlichen Behandlungsvorrichtung,
- Fig. 2: zeigt eine vergrößerte Darstellung der Frontseite des in Fig. 1 dargestellten Steuergeräts,
- Fig. 3a: zeigt beispielhaft den Aufbau der in dem in Fig. 1 gezeigten Steuergerät gespeicherten Datenbank,
- Fig. 3b und 3c: zeigen die Kopplung eines Antriebsmotors mit einem dentalen Handstück bzw. Winkelstück sowie einem Behandlungswerkzeug,
- Fig. 4: zeigt ein schematisches Blockdiagramm zur Erläuterung der Erstellung eines benutzerspezifischen Behandlungs- bzw. Betriebsprogramms,
- Fig. 5: zeigt beispielhaft herstellerseitig vorgegebene Betriebsprogramme,
- Fig. 6: zeigt ein Ausführungsbeispiel einer einteiligen Ablage für Behandlungsinstrumente, Behandlungswerkzeuge sowie den Antriebsmotor, die bei der erfindungsgemäßen ärztlichen Behandlungsvorrichtung zum Einsatz kommen kann,
- Fig. 7: zeigt verschiedene Ansichten einer zweiteiligen Variante der in Fig. 6 gezeigten Ablage,
- Fig. 8: zeigt ein erstes Ausführungsbeispiel einer Einrichtung zur Drehmomentkalibrierung eines mit dem Antriebsmotor gekoppelten Behandlungsinstruments,
- Fig. 9: zeigt verschiedene Ansichten eines zweiten Ausführungsbeispiels einer Einrichtung zur Drehmomentkalibrierung, und
- Fig. 10: zeigt die Kennlinie des Drehmoments gegenüber dem Antriebsmotorstrom zur Erläuterung der Drehmomentkalibrierung mit der in Fig. 8 bzw. 9 gezeigten Einrichtung.

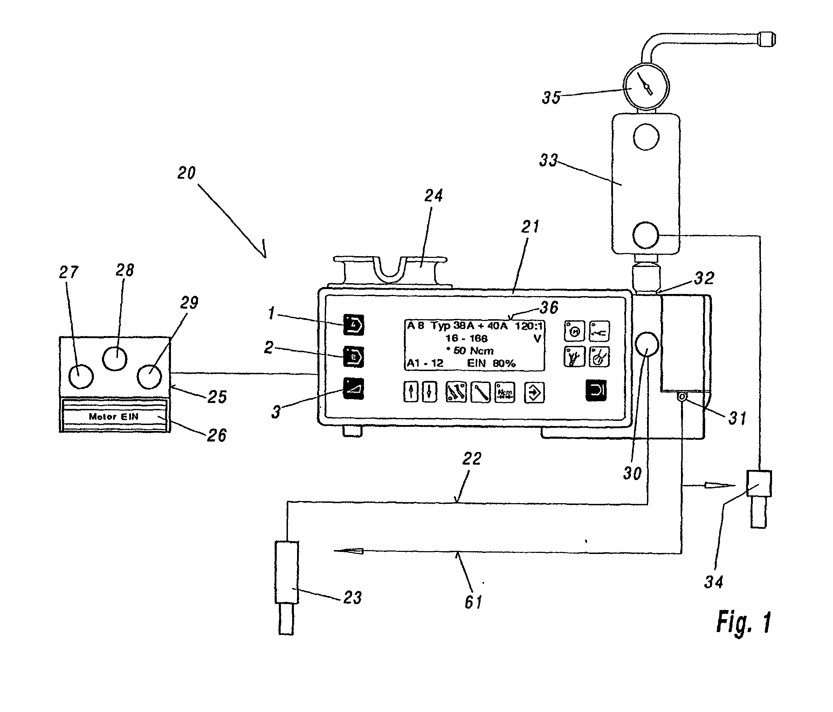

Fig. 1 zeigt ein bevorzugte Ausführungsbeispiel einer zahnärztlichen Behandlungsvorrichtung 20, wobei es sich insbesondere um eine zahnärztliche Behandlungsvorrichtung zum Einsatz in der Mikrochirurgie, HNO-Chirurgie, plastischen Chirurgie, Kieferchirurgie und Implantologie handelt.

Der zentrale Bestandteil der in Fig. 1 dargestellten ärztlichen Behandlungsvorrichtung ist ein Steuergerät 21, welches abhängig von vorgegebenen Betriebsparameterinformationen einen Antriebsmotor steuert, der mit einem Behandlungsinstrument sowie einem daran gekoppelten Behandlungswerkzeug koppelbar ist. Gemäß Fig. 1 ist der Antriebsmotor 23 über eine Versorgungsleitung 22 an einen entsprechenden Anschluß 30 angeschlossen. Die Versorgungsleitung 22 kann durch eine Schnellkupplung an dem Steuergerät, d.h. dem Anschluß 30, sowie durch eine Schraubkupplung an dem Antriebsmotor 23 befestigt sein. Als Antriebsmotor 23 wird vorzugsweise ein kollektorloser Motor mit Stellungsmelder verwendet, dessen Drehzahlbereich zwischen 200 und 40 000 Umdrehungen/min. liegt. Das Drehmoment dieses Antriebsmotors beträgt beispielsweise 5 Ncm bei einer Antriebsdrehzahl von 200/min. Auf den Antriebsmotor 23 kann - wie bereits erwähnt worden ist - ein Behandlungsinstrument, wie z.B. ein zahnärztliches Handstück oder ein Reduzierwinkelstück, aufgesteckt werden, welches das eigentliche Behandlungswerkzeug, wie z.B. einen Bohrer od.dgl. trägt. Abhängig von der Drehzahlübertragung des mit dem Antriebsmotor gekoppelten Behandlungsinstruments können somit unterschiedliche Drehzahlbereiche abgedeckt und unterschiedliche maximale Drehmomentwerte erzielt werden.

Bei einer chirurgischen Behandlung wird in der Regel sowohl der Antriebsmotor als auch das Behandlungsinstrument und das Behandlungswerkzeug mit von dem behandelten Körper stammenden Krankheitserregern kontaminiert. Aus diesem Grund ist es erforderlich, daß sowohl der Antriebsmotor 23 als auch die Versorgungsleitung 22 autoklavierbar und somit leicht sterilisierbar sind.

Für bestimmte Behandlungsarten, bei denen eine Wärmeentwicklung an der Behandlungsstelle auftritt, ist die Zufuhr eines Kühlmittels erforderlich. Aus diesem Grund weist die in Fig. 1 gezeigte ärztliche Behandlungsvorrichtung eine Kühlmittelpumpe auf, die über einen entsprechenden Anschluß 31 ein Kühlmittel, wie z.B. eine NaCl-Salzlösung, der Behandlungsstelle zuführt. Die an den Anschluß 31 angeschlossene Kühlmittelleitung 61 kann extern zusammen mit der Versorgungsleitung 22 dem Antriebsmotor 23 bzw. dem daran angekoppelten Behandlungsinstrument zugeführt werden. Alternativ kann jedoch die Kühlmittelleitung 61 auch zumindest teilweise in die Versorgungsleitung 22 bzw. das Antriebsmotor-Handstück 23 integriert sein. Wie nachfolgend noch näher beschrieben wird, steuert das Steuergerät 21 der ärztlichen Behandlungsvorrichtung 20 die Zufuhr des Kühlmittels über die Kühlmittelleitung 61 und regelt insbesondere die zugeführte Kühlmittelmenge.

Die in Fig. 1 gezeigte ärztliche Behandlungsvorrichtung 20 kann wahlweise auch mit zwei Anschlüssen 30 und zwei Antriebsmotoren 23 ausgestaltet sein, die insbesondere unterschiedliche Drehzahlbereiche oder Drehmomentwerte aufweisen, um somit unterschiedliche Anwendungsgebiet abdecken zu können. Die beiden Antriebsmotore können unabhängig voneinander betrieben werden. Vorzugsweise ist dem zweiten Antriebsmotor eine zweite Kühlmittelpumpe zugeordnet, die analog zu der zuvor beschriebenen Kühlmittelpumpe mit der entsprechenden Kühlmittelleitung 61 ausgestaltete sein kann.

Eine Besonderheit der in Fig. 1 gezeigten ärztlichen Behandlungsvorrichtung 20 ist die Verwendung einer PneumatikPneumatik-Antriebseinrichtung 33, die an einen Luftanschluß 32 des Steuergeräts angeschlossen ist. Diese Pneumatik-Antriebseinrichtung 33 ist zum Betreiben bestimmter Sonder-Behandlungsinstrumente vorgesehen, die zum einen Luftantrieb und zum anderen eine sterile Kühlflüssigkeit erfordern und an einen Multiflex-Anschluß 34 angeschlossen werden. Bei diesen Sonder-Behandlungsinstrumenten kann es sich um bestimmte Instrumente für die Paradontologie, Kronen- und Brückenabnahme, Endodontie und Wurzelspitzenresektion handeln. Der von der Pneumatik-Antriebseinrichtung 33 gelieferte Antriebsluftdruck kann mit Hilfe der Pneumatik-Antriebseinrichtung 33 sowie einem entsprechend vorgesehenen Luftdruckmesser 35 überwacht und beliebig reguliert und eingestellt werden. Des weiteren kann mit Hilfe des Steuergeräts 21 der Luftdruck sowie die zugeführte Kühlflüssigkeitsmenge der Pneumatik-Antriebseinrichtung 33 gesteuert werden.

Wie aus Fig. 1 ersichtlich ist, weist die Frontseite der Steuereinrichtung 21 eine integrierte Anzeigeneinheit 36 auf, welche - wie noch nachfolgend ausführlicher zu beschreiben ist - die augenblicklich ausgewählten bzw. geltenden Betriebsparameterinformationen anzeigt. Da bei Aktivierung der Pneumatik-Antriebseinrichtung 33 der Antrieb des daran angeschlossenen SonderSonder-Behandlungsinstruments 34 durch Luftdruck erfolgt, kann die Anzeigeneinheit 36 in diesem Fall die geltenden Luftdruckeinstellungen der Pneumatik-Antriebseinrichtung 33 zusammen mit der geltenden Kühlmitteleinstellung anzeigen. Ebenso ist denkbar, daß auf der Anzeigeneinheit 36 in diesem Fall ausschließlich die Kühlmitteldaten wiedergegeben werden.

Des weiteren ist gemäß Fig. 1 eine nachfolgend noch näher zu beschreibende Ablage 24 vorgesehen; die zur Ablage von Behandlungswerkzeugen, Behandlungsinstrumenten sowie des Antriebsmotors 23 vorgesehen ist. Bei der Ablage 24 handelt es sich um eine separate Multifunktionsablage, in die der Antriebsmotor 23 mit der dazugehörigen Versorgungsleitung 22 bzw. dem Versorgungsschlauch sowie benutzte Behandlungsinstrumente und Behandlungswerkzeuge abgelegt werden können. Wie nachfolgend noch näher beschrieben werden wird, kann die Ablage 24 sterilisiert werden und dient zugleich beim Sterilisieren als Verpackung für den darin abgelegten Antriebsmotor 23, das Behandlungsinstrument sowie den Versorgungsschlauch 22.

Es ist bereits bekannt, das Steuergerät 21 der ärztlichen Behandlungsvorrichtung 20 mit Hilfe eines Fußanlassers 25 zu betreiben, um eine manuelle Betätigung der Funktionstasten des Steuergeräts 21 während der Behandlung zu vermeiden (vgl. z.B. Druckschrift EP - A2 - 0525 539). Aus der JP-A-62- 16106 ist ein Fußschalter zur drahtlosen Fernsteuerung eines zahnmedizinischen Stuhls bekannt. Dieses Prinzip ist bei der in Fig. 1 dargestellten ärztlichen Behandlungsvorrichtung angewendet, d.h. der Fußschalter 25 kommuniziert mit einer entsprechend in dem Steuergerät 21 vorgesehenen Empfangseinheit über eine kabellose Übertragungsstrecke, die z.B. durch eine Funk- oder Infrarot-Übertragungsstrecke gebildet sein kann. Der Fußschalter 25 weist demnach eine selbständige Energieversorgung in Form eines Akkumulators oder einer Batterie auf. Wie nachfolgend noch beschrieben wird, kann mit Hilfe der in Fig. 1 dargestellten ärztlichen Behandlungsvorrichtung zwischen hersteller- bzw. werkseitig vorgegebenen Betriebsprogrammen, die nicht von einem Benutzer veränderbar sind, und benutzerspezifisch erstellten und abgespeicherten Betriebsprogrammen ausgewählt werden. Nach Auswahl der herstellerseitigen Betriebsprogrammebene bzw. der benutzerspezifischen Betriebsprogrammebene an dem Steuergerät 21 kann mit Hilfe des Schalters 28 der Benutzer über den Fußschalter 25 sämtliche Betriebsprogramme der ausgewählten Betriebsprogrammebene durchschalten und auf diese Weise ein gewünschtes Betriebsprogramm dieser Betriebsprogrammebene auswählen. Dies bedeutet, daß das Steuergerät nicht mit den (möglicherweise unsterilen) Fingern berührt und bedient werden muß, da die gesamte Steuerung über den Fußschalter 25 erfolgen kann. Alternativ kann der Fußschalter 25 auch einen Auswahlschalter zum Umschalten zwischen der herstellerseitigen, unveränderbaren Betriebsprogrammebene und der benutzerspezifischen, beliebig veränderbaren Betriebsprogrammebene aufweisen, so daß auch die Auswahl zwischen diesen beiden Betriebsprogrammebenen durch den Fußschalter 25 erfolgen kann. Über die Schalter 26, 27 und 29 kann der Benutzer manuell in das ausgewählte Betriebsprogramm eingreifen, wobei der Schalter 26 zur direkten Ansteuerung des Antriebsmotors 23 dient. Dieser Schalter 26 besitzt die Funktion eines Gaspedals und dient zum Ein- und Ausschalten des Antriebsmotors 23. Ebenso ist denkbar, daß über den Schalter 26 abhängig von dem Niederdrücken des Schalters 26 die Drehzahl des Antriebsmotors 23 eingestellt wird. Mit Hilfe des Schalters 27 kann die Kühlmittelpumpe 31 ein- und ausgeschaltet werden. Schließlich kann mit Hilfe des Schalters 29 der (standardmäßig) vorgegebene Rechtslauf des Antriebsmotors 23 auf Linkslauf und umgekehrt umgestellt werden. Vorteilhafterweise ist der Fußschalter 25 wasserdicht ausgestaltet, um stets die Funktionsfähigkeit des Fußschalters 25 zu gewährleisten.

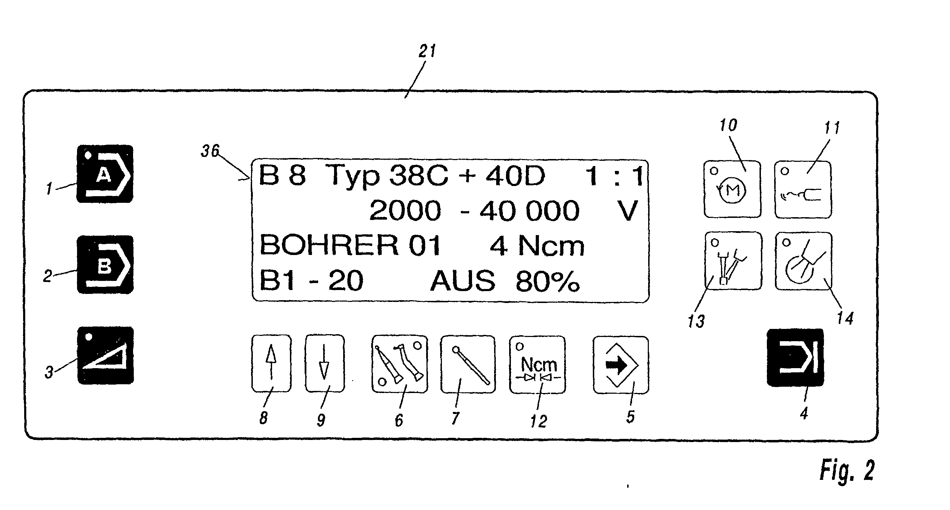

Nach der allgemeinen Beschreibung der Bestandteile der in Fig. 1 dargestellten ärztlichen Behandlungsvorrichtung soll nachfolgend unter Bezugnahme auf Fig. 2-5 die Funktionsweise dieser ärztlichen Behandlungsvorrichtung näher beschrieben werden.

Fig. 2 zeigt eine vergrößerte Darstellung der Frontseite des in Fig. 1 dargestellten Steuergerätes 21 der ärztlichen Behandlungsvorrichtung 20. In die Frontseite ist eine Anzeigeneinheit 36, z.B. eine Flüssigkristallanzeige integriert, die vorzugsweise vierzeilig die geltenden bzw. ausgewählten Betriebsparameterinformationen anzeigt, so daß eine optische Überwachung des Betriebs der ärztlichen Behandlungsvorrichtung möglich ist. Des weiteren weist die Frontseite des Steuergeräts 21 eine Vielzahl von Auswahltasten 1 - 14 auf, bei denen es sich insbesondere aus hygienischen Gründen um Folientasten handeln kann, die durch einen kurzen Tastendruck aktivierbar sind. Jeder dieser Tasten 1-14 ist eine bestimmte Funktion zugewiesen, die nachfolgend näher erläutert werden soll.

Die in Fig. 1 dargestellte ärztliche Behandlungsvorrichtung besitzt - wie bereits zuvor beschrieben worden ist - im wesentlichen zwei Programmebenen, wobei in der einen Programmebene aus herstellerseitigen Betriebsprogrammen zum Betreiben bestimmter Behandlungsinstrumente und Behandlungswerkzeuge ausgewählt werden kann. Diese werk- bzw. herstellerseitige Programmebene umfaßt ausschließlich Betriebsprogramme, die von einem Benutzer nicht verändert, d.h. nicht überschrieben werden können. Des weiteren ist eine benutzerspezifische Betriebsprogrammebene vorgesehen, die eine Vielzahl von benutzerspezifischen Betriebsprogrammen umfaßt, die zuvor von einem Benutzer erstellt und von diesem beliebig veränderbar sind. Die herstellerseitig vorgegebenen Betriebsprogramme sowie die benutzerspezifisch erstellten Betriebsprogramme werden in einem Speicher des Steuergeräts 21 abgelegt. Im Prinzip kann eine beliebige Anzahl von herstellerseitig vorgegebenen Betriebsprogrammen oder benutzerspezifischen Betriebsprogrammen gespeichert werden. Aus Gründen der Übersichtlichkeit und des Speicherplatzes wird jedoch vorteilhafterweise die maximal zulässige Anzahl von herstellerseitig vorgegebenen bzw. benutzerspezifisch erstellten Betriebsprogrammen beispielsweise auf jeweils 100 Betriebsprogramme begrenzt.

Nach Auswahl eines Behandlungs- bzw. Betriebsprogramms aus diesen beiden Programmebenen werden die entsprechenden Betriebsparameterinformationen des ausgewählten Betriebsprogramms auf der Anzeigeneinheit 36 dargestellt und der Antriebsmotor 23 kann gemäß diesen vorgegebenen Betriebsparameterinformationen durch das Steuergerät 21 ggfs. in Zusammenwirkung mit dem Fußschalter 25 angesteuert werden.

Neben diesen beiden Programmebenen ist bei der in Fig. 1 gezeigten ärztlichen Behandlungsvorrichtung auch ein Einstellbetrieb vorgesehen, in dem ein Benutzer nach Auswahl eines Betriebsprogramms manuell die dem Betriebsprogramm entsprechenden Betriebsparameterwerte verändern kann. Ebenso kann der Benutzer auch ohne vorherige Auswahl eines Betriebsprogramms die Betriebsparameter mit Hilfe der Funktionstasten des Steuergeräts 21 bzw. des Fußschalters 25 einstellen. In diesem Einstellmodus arbeitet der Benutzer vorwiegend mit Erfahrungs- bzw. Gefühlswerten.

Mit Hilfe der in Fig. 2 dargestellten Taste 1 kann ein Benutzer zwischen sämtlichen herstellerseitig vorgegebenen Betriebsprogrammen, die nicht von dem Benutzer verändert werden können, auswählen. Dabei kann vorgesehen sein, daß mit jeder Betätigung der Taste 1 das nächste herstellerseitig vorgegebene Betriebsprogramm ausgewählt und in der Anzeigeneinheit 36 angezeigt wird. In diesem Fall können sämtliche herstellerseitig vorgegebenen Betriebsprogramme lediglich durch Betätigung der Taste 1 durchblättert werden. Alternativ kann vorgesehen sein, daß durch Betätigung der Taste 1 zunächst in die herstellerseitig vorgegebene Programmebene gewechselt wird, in der dann anschließend mit Hilfe der Tasten 8 und 9 die einzelnen herstellerseitig vorgegebenen Betriebsprogramme durchblättert werden können. Auch in diesem Fall wird das jeweils geltende bzw. ausgewählte Betriebsprogramm bzw. die entsprechenden Betriebsparameterinformationen in der Anzeigeneinheit 36 dargestellt.

Mit Hilfe der Taste 2 kann analog zu der Taste 1 zwischen sämtlichen benutzerspezifisch erstellten Behandlungs- bzw. Betriebsprogrammen ausgewählt werden, wobei das Auswählen eines benutzerspezifisch erstellten Betriebsprogramms entweder allein durch Betätigen der Taste 2 oder in Kombination mit den Tasten 8 und 9 erfolgen kann. Auch die einem ausgewählten benutzerspezifisch erstellten Betriebsprogramm entsprechenden Betriebsparameterinformationen werden in der Anzeigeneinheit 36 dargestellt.

Wie bereits beschrieben worden ist, wird jedes Betriebsprogramm durch bestimmte Betriebsparameterinformationen definiert, die insbesondere den Typ des verwendeten Behandlungsinstruments, den Typ des verwendeten Behandlungswerkzeugs, technische Informationen zum Betrieb des Behandlungsinstruments bzw. des Behandlungswerkzeugs sowie Kühlmittelinformationen umfassen.

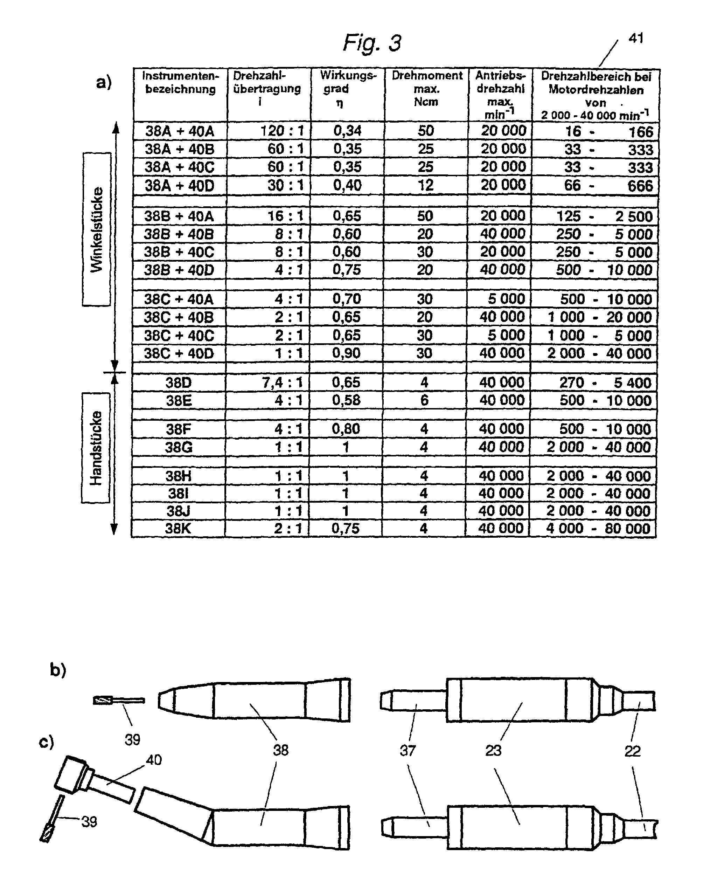

Wie in Fig. 3b und 3c gezeigt ist, wird im Falle einer zahnärztlichen Behandlungsvorrichtung auf die Antriebswelle 37 des über den Versorgungsschlauch 22 an das Steuergerät 21 angeschlossenen Antriebsmotors ein Behandlungsinstrument 38 aufgesetzt, wobei es sich gemäß Fig. 3b um ein Handstück und gemäß Fig. 3c um ein Winkelstück handelt. Ein Behandlungswerkzeug 39 wird mit dem aufgesetzten Behandlungsinstrument 38 gekoppelt, wobei im Falle eines in Fig. 3c dargestellten Winkelstücks 38 auf das Winkelstück 38 zudem ein Drehzahlübertragungskopf 40 mit ggfs. vorhandener Drehzahluntersetzung aufgesetzt werden kann, in den anschließend das Behandlungswerkzeug 39 eingeführt wird. Abhängig von dem Anwendungsgebiet ist eine Vielzahl von Behandlungsinstrumenten 38, Kopfteilen 40 und Behandlungswerkzeugen 39 bekannt. Während sich die Behandlungsinstrumente 38 bzw. Kopfteile 40 vorwiegend in der Drehzahlübertragung bzw. Drehzahluntersetzung unterscheiden, kann es sich bei den Behandlungswerkzeugen 39 beispielsweise um Körner, Bohrer, Fräser oder Gewindeschneider usw. handeln.

In dem Steuergerät 21 ist eine Datenbank vorhanden, die Informationen zu sämtlichen verfügbaren Behandlungsinstrumenten 38, Kopfteilen 40, Behandlungswerkzeugen 39 sowie den verfügbaren Kühlmittelmöglichkeiten enthält. Der die Behandlungsinstrumente 38 betreffende Datensatz 41 dieser Datenbank ist beispielhaft in Fig. 3a dargestellt. Daraus ist ersichtlich, daß der die Behandlungsinstrumente 38 betreffende Datensatz beispielsweise die Typ- bzw. Produktbezeichnung für verschiedene Winkelstücke 38A-C oder Handstücke 38D-K beinhaltet. Wird als Behandlungsinstrument 38 ein Winkelstück (Typ 38A-38C) eingesetzt, umfaßt die erste Spalte des in Fig. 3a gezeigten Datensatzes jeweils die Kombination eines Winkelstücks 38A-C mit einem entsprechenden Kopfteil 40A-D. Zu jedem Behandlungsinstrument bzw. zu jeder Kombination eines Winkelstücks mit einem Kopfteil umfaßt der in Fig. 3a gezeigte Datensatz zudem die sich daraus ergebenden technischen Eigenschaften, wie z.B. die daraus resultierende Drehzahlübertragung, der Wirkungsgrad, das maximale Drehmoment, die maximal zulässige Antriebsdrehzahl sowie der sich bei einer Antriebsdrehzahl des Antriebsmotors 23 von 2000 - 40 000 /min ergebende Drehzahlbereich. Es können theortetisch beliebig viele dieser Hand- und Winkelstückdatensätze in der Datenbank abgelegt werden.

Neben dem in Fig. 3a gezeigten behandlungsinstrumentenbezogenen Datensatz 41 umfaßt - wie bereits beschrieben worden ist - die Datenbank zudem einen behandlungswerkzeugbezogenen Datensatz sowie einen kühlmittelbezogenen Datensatz. Der behandlungswerkzeugbezogene Datensatz beschreibt beispielsweise für die einzelnen Typen von verfügbaren Behandlungswerkzeuge, wie z.B. Bohrer, die dazu gehörigen technischen Eigenschaften. Der kühlmittelbezogene Datensatz beschreibt die Kühlmittelmöglichkeiten, wie z.B. die einstellbaren Kühlmittelmengen oder Kühlmittelpumpenleistungen.

Diese drei zuvor beschriebenen Datensätze der in dem Steuergerät 21 abgelegten Datenbank können nunmehr in Einheiten als Betriebsprogramme zusammengefaßt werden. Diese Betriebsprogramme stellen somit jeweils eine Kombination eines bestimmten Behandlungsinstrumentes mit einem bestimmten Behandlungswerkzeug dar, wobei für diese Kombination bestimmte technische Betriebsparameter, wie z.B. das Drehmoment oder der Drehzahlbereich, sowie eine entsprechende Kühlmitteleinstellung vorgegeben sein kann. Somit können in der ersten und zweiten Programmebene für verschiedene Anwendungen unterschiedliche Betriebsprogramme vorgesehen sein.

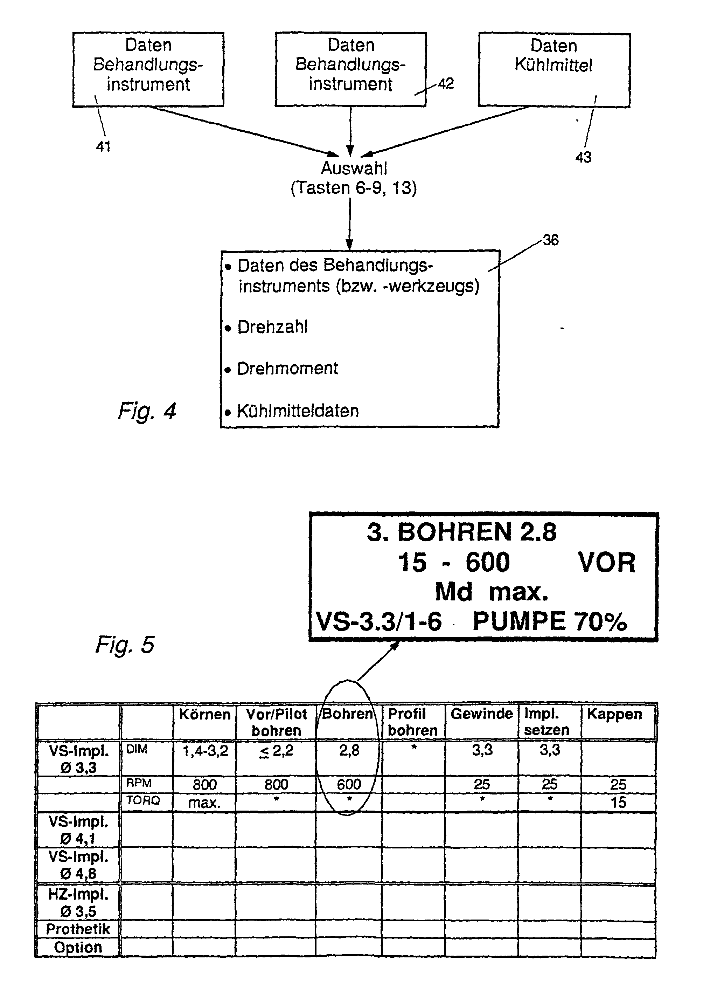

So zeigt Fig. 5 beispielsweise anhand der herstellerseitig vorgegebenen Programmebene, die von dem Benutzer nicht veränderbare Betriebsprogramme umfaßt, abhängig für in der ersten Spalte dargestellte Anwendungen unterschiedliche Betriebsprogrammfolgen. Dabei ist in der ersten Zeile in Fig. 5 beispielhaft das Setzen eines Implantats mit einem Durchmesser von 3,3 mm dargestellt. Diese Anwendung umfaßt gemäß Fig. 5 insgesamt sieben unterschiedliche entsprechend zugeordnete Betriebsprogramme, die mit "Körnen", "Vor/Pilotbohren", "Bohren", "Profilbohren", "Gewinde", "Implantatsetzen" und "Kappen" bezeichnet sind. Jedes dieser Betriebsprogramme umfaßt Informationen über den dabei verwendeten Bohrerdurchmesser ("DIM"), die benutzte Betriebsdrehzahl ("RPM") sowie das zulässige Drehmoment ("TORQ"). Der Benutzer kann somit abhängig von der gewählten Anwendung nacheinander die dieser Anwendung zugeordneten Betriebsprogramme durchlaufen bzw. diese auswählen, wobei nach Auswahl eines entsprechenden Betriebsprogramms die diesem Betriebsprogramm zugeordnete Betriebsparameterinformationen auf der Anzeigeneinheit 36 des Steuergerätes 21 dargestellt werden. Dies ist in Fig. 5 beispielhaft anhand des Betriebsprogramms "Bohren" angedeutet. In der ersten Zeile der Anzeigeneinheit 36 können Informationen über das verwendete Behandlungsinstrument bzw. Behandlungswerkzeug dargestellt sein. Gemäß Fig. 5 enthält die erste Zeile der Anzeigeneinheit 36 zudem mit der Bezeichnung "3." eine Information über die Sequenznummer des augenblicklich ausgewählten Betriebsprogramms der entsprechenden Anwendung. Die zweite Zeile der Anzeigeneinheit 36 umfaßt gemäß Fig. 5 Informationen über den dem Betriebsprogramm zugewiesenen Drehzahlbereich sowie den eingestellten Vorwärtsbetrieb ("VOR"). In der dritten Zeile wird das maximale Drehmoment angegeben, während die vierte Zeile gemäß Fig. 5 Informationen über die zugeführte Kühlmittelmenge (gemäß Fig. 5 70 %) und die Bezeichnung der dem ausgewählten Betriebsprogramm entsprechenden Anwendung enthalten kann. Selbstverständlich kann die Anzeigeneinheit 36 auch eine Zeilenanzahl größer als vier aufweisen und die dem ausgewählten Betriebsprogramm entsprechenden Betriebsparameterinformationen auch in anderer Form sowie anderer Reihenfolge darstellen.

Der Aufbau der Betriebsprogramme wurde zuvor anhand der unveränderbar herstellerseitig vorgegebenen Betriebsprogramme erläutert. Dieser Aufbau trifft jedoch genauso auf die benutzerspezifisch erstellten Betriebsprogramme zu. Wie bereits zuvor beschrieben worden ist, kann mit Hilfe der in Fig. 2 dargestellten Taste 2 sowie ggfs. in Kombination mit den Tasten 8 und 9 ein beliebiges benutzerspezifisch erstelltes Betriebsprogramm ausgewählt werden. Auch die in der zweiten Programmebene abgelegten benutzerspezifischen Betriebsprogramme umfassen demnach Informationen über den zu benutzenden Behandlungsinstrumententyp, ggfs. Kopfteiltyp, Behandlungswerkzeugtyp sowie die entsprechenden technischen Betriebsparameter, wie z.B. Drehzahlbereich oder Drehmoment. Bevor ein beliebiges benutzerspezifisch erstelltes Betriebsprogramm jedoch mit Hilfe der Taste 2 ausgewählt werden kann, muß zumindest ein derartiges benutzerspezifisches Betriebsprogramm erstellt worden sein. Dies soll nachfolgend unter Bezugnahme auf Fig. 4 näher erläutert werden.

Wie bereits zuvor beschrieben worden ist, kann mit Hilfe der beiden Tasten 1 und 2 ein beliebiges Betriebsprogramm der herstellerseitig vorgegebenen, unveränderbaren ersten Programmebene bzw. der benutzerspezifisch erstellten, beliebig veränderbaren zweiten Programmebene ausgewählt werden. Zudem ist ein Einstellmodus vorgesehen, der durch die in Fig. 2 dargestellte Taste 2 aufgerufen werden kann. In diesem Einstellmodus können sämtliche technische Daten durch den Benutzer frei eingestellt werden. Nach Betätigen der Taste 3 erscheint beispielsweise in der ersten Zeile der in Fig. 2 gezeigten Anzeigeeinheit 36 lediglich die Bezeichnung "frei", während in der zweiten bis vierten Zeile Informationen über die augenblicklich eingestellte Istdrehzahl des Antriebsmotors, den Drehmomentbereich des Antriebsmotors sowie den Betriebszustand der Kühlung angegeben werden. In diesem Einstellmodus arbeitet der Benutzer vorwiegend aufgrund seiner Erfahrungs- und Gefühlswerte, ohne daß ihm bestimmte Betriebsparameter eines gespeicherten Betriebsprogramms vorgegeben sind.

Des weiteren kann der Benutzer über die Taste 6 eine bestimmte Aufsatzkombination des Antriebsmotors einstellen. Dabei wird nach Betätigung der Taste 6 der beispielhaft in Fig. 3a dargestellte instrumentenbezogene Datensatz 41 der intern abgelegten Datenbank des Steuergeräts aufgerufen, wobei der Benutzer mit Hilfe der Tasten 8 und 9 durch die einzelnen Datensatzeinträge blättern kann. Der jeweils augenblicklich ausgewählte Datensatzeintrag wird dabei beispielsweise in der ersten Zeile der Anzeigeneinheit 36 dargestellt. Ebenso kann der Benutzer mit Hilfe der Taste 7 den behandlungswerkzeugbezogenen Datensatz aufrufen und wiederum mit Hilfe der Tasten 8 und 9 durch die einzelnen Datensatzeinträge blättern, wobei die jeweils gültige Auswahl ebenfalls auf der Anzeigeneinheit 36 dargestellt wird. Schließlich kann der Benutzer auch mit Hilfe der Taste 13 den vorgegebenen, in dem Steuergerät 21 abgelegten kühlmittelbezogenen Datensatz aufrufen und mit Hilfe der Tasten 8 und 9 die gewünschte Kühlmittelmenge bzw. Kühlmittelzufuhrleistung der Kühlmittelpumpe auswählen und einstellen, wobei die jeweils gültigen Kühlmitteldaten auch auf der Anzeigeneinheit 36 dargestellt werden. Des weiteren kann mit Hilfe der Taste 13 die Kühlmittelzufuhr einund ausgeschaltet werden.

Wie in Fig. 4 gezeigt ist, erfolgt somit mit Hilfe der Tasten 6-9 und 13 eine benutzerspezifische Auswahl der einzelnen Einträge des behandlungsinstrumentenbezogenen Datensatzes 41, des behandlungswerkzeugbezogenen Datensatzes 42 sowie des kühlmittelbezogenen Datensatzes 43, wobei die sich daraus ergebende Kombination der einzelnen Einstellungen auf der Anzeigeneinheit 36 dargestellt wird.

Durch Betätigen der Taste 5 kann schließlich der Benutzer diese Kombination als ein neues benutzerspezifisch erstelltes Behandlungs- bzw. Betriebsprogramm in der zu der Taste 2 gehörenden benutzerspezifischen Programmebene ablegen. Nach Betätigen der Taste 5 wird das neu erstellte benutzerspezifische Betriebsprogramm auf den nächsten freien Speicherplatz der benutzerspezifischen Programmebene abgelegt. Sollten sämtliche Speicherplätze dieser zweiten benutzerspezifischen Programmebene belegt sein, kann ein Warnton ausgegeben werden oder das neue Betriebsprogramm automatisch unter den ersten Speicherplatz der zweiten Programmebene abgelegt werden, wobei jedoch in diesem Fall das zuvor unter dem ersten Speicherplatz abgelegte Betriebsprogramm verlorengeht.

Neben der zuvor beschriebenen Vorgehensweise zur Erstellung eines benutzerspezifischen Betriebsprogramms kann der Benutzer auch ein gespeichertes Betriebsprogramm der den Auswahltasten 1 und 2 entsprechenden Programmebenen auswählen, mit Hilfe der Tasten 6 - 9 und 13 verändern und als neues benutzerspezifisches Betriebsprogramm abspeichern, wobei jedoch nur die unter der Taste 2 abgelegten benutzerspezifischen Betriebsprogramme überschrieben werden können.

Wie bereits zuvor erläutert worden ist, werden die einem ausgewählten Betriebsprogramm entsprechenden Betriebsparameterinformationen auf der Anzeigeneinheit 36 dargestellt. Dabei ist in Fig. 2 beispielhaft die Auswahl eines benutzerspezifischen Betriebsprogramms mit Hilfe der Taste 2 dargestellt. Wie in Fig. 2 gezeigt ist, gibt die erste Zeile der Anzeigeneinheit 36 nach Auswahl eines benutzerspezifischen Betriebsprogramms zunächst die Bezeichnung des Betriebsprogramms ("B 8") sowie den dem ausgewählten Betriebsprogramm entsprechenden Behandlungsinstrumententyp mit der sich daraus ergebenden Drehzahlübertragung wieder (vgl. auch Fig. 3a). Wie bereits anhand Fig. 3a beschrieben worden ist, handelt es sich in diesem Fall um die Kombination eines Winkelstücks vom Typ "38C" mit einem Kopfteil vom Typ "40D". In der zweiten Zeile ist der Drehzahlbereich angegeben, wobei als Betriebsart der Vorwärtsbetrieb ("V") festgelegt ist. In der dritten Zeile ist der Typ des zu verwendenden Behandlungswerkzeugs ("Bohrer 01") und das maximale Drehmoment dargestellt. In der vierten Zeile ist schließlich die Gesamtzahl der gespeicherten benutzerspezifischen Betriebsprogramme ("B 1 - 20") zusammen mit kühlmittelbezogenen Daten dargestellt. Gemäß der vierten Zeile beträgt die eingestellte Kühlmittelmenge 80 %, wobei jedoch die Kühlmittelzufuhr ausgeschaltet ist.

Nachfolgend sollen unter Bezugnahme auf Fig. 2 die restlichen Funktionstasten des Steuergerätes 21 beschrieben werden. Mit Hilfe der Taste 10 kann die Drehrichtung des Antriebsmotors von Vorwärtsbetrieb auf Rückwärtsbetrieb und umgekehrt umgeschaltet werden. Der Antriebsmotor weist zu der jeweils gewählten drehmomentbegrenzten Drehrichtung ein in die entgegengesetzte Drehrichtung erhöhtes Drehmoment auf. Durch die Taste 11 kann der in Fig. 1 dargestellte Pneumatik-Anschluß 32 aktiviert und deaktiviert werden. Durch Betätigung der Taste 12 kann eine nachfolgend noch näher zu beschreibende Drehmomentkalibrierungsprozedur aufgerufen werden, die zur Drehmomentkalibrierung des Antreibsmotors mit einem darauf aufgesetzten Behandlungsinstrument dient. Die Taste 13 dient - wie bereits beschrieben worden ist - zum Ein-/Ausschalten der Kühlmittelzufuhr, wobei in Kombination mit den Tasten 8 und 9 die Kühlmittelmenge eingestellt werden kann. Um Salzablagerungen infolge der NaCl-Salzlösung in der in Fig. 1 gezeigten Kühlmittelleitung 61 sowie der Sterilisation zu vermeiden, muß die Kühlmittelleitung 61 in regelmäßigen Abständen mit destilliertem Wasser durchgespült werden. Diese Spülung der Kühlmittelleitung kann durch Betätigung der Taste 4 ausgelöst werden, wobei die Spülung vorzugsweise nur gestartet wird, wenn tatsächlich zuvor Kühlmittel verwendet worden ist. Mit der Taste 4 können schließlich die einzelnen ausgewählten Betriebsprogramme, d.h. die Behandlung eines Patienten, beendet werden, wobei vorzugsweise nach Betätigung dieser Taste 4 auf der Anzeigeneinheit 36 Pflegehinweise sowie weitere hilfreiche Informationen angezeigt werden.

Zudem kann die Anzeigeeinheit 36 zur Anzeige von Fehlermeldungen dienen, die über während des Betriebs der ärztlichen Behandlungsvorrichtung 20 bzw. des Steuergeräts 21 auftretende Fehler informieren. So kann beispielsweise auf Fehler in der Elektronik des Steuergeräts 21, auf Fehler des Antriebsmotors 23 oder auf die Datenbank des Steuergeräts 21 betreffende Fehler, wenn z.B. sämtliche Speicherplätze der beiden den Tasten 1 und 2 zugeordneten Programmebenen belegt sind, hingewiesen werden. Ebenso ist denkbar, das Steuergerät 21 mit einer automatischen Behandlungstyp-Erkennungseinrichtung auszustatten, so daß eine Abweichung zwischen dem einem ausgewählten Betriebsprogramm entsprechenden Behandlungsinstrumententyp und dem tatsächlich auf den Antriebsmotor 23 aufgesteckten Behandlungsinstrumententyp erkannt und eine entsprechende Fehlermeldung ausgegeben werden kann. Derartige Behandlungsinstrumententyp-Erkennungseinrichtungen sind bereits bekannt.

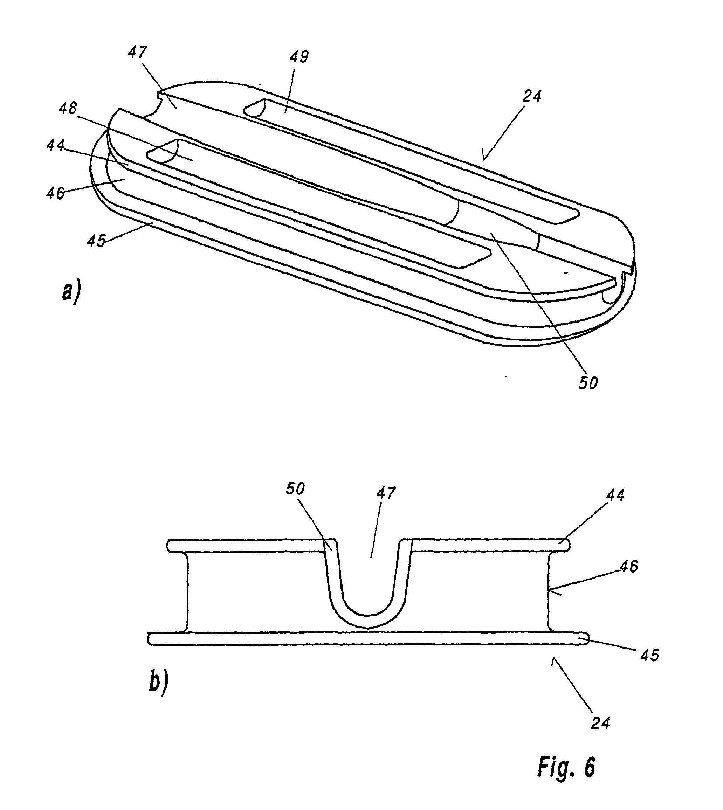

Fig. 6 zeigt ein erstes Ausführungsbeispiel der in Fig. 1 dargestellten Ablage 24, die zur Aufnahme des Antriebsmotors 23 sowie von Behandlungsinstrumenten und Behandlungswerkzeugen und dem Versorgungsschlauch 22 dient, wobei Fig. 6a eine perspektivische Ansicht dieses ersten Ausführungsbeispiels und Fig. 6b eine Frontalansicht dieses Ausführungsbeispiels darstellt.

Die in Fig. 6 dargestellte Ablage 24 besitzt eine sich in Längsrichtung der Ablage 24 erstreckende durchlaufende Vertiefung 47, die zur Aufnahme des in Fig. 1 dargestellten Antriebsmotors 23 dient. Die Vertiefung 47 ist im wesentlichen an die Außenform des Antriebsmotors 23 angepaßt und weist an ihrem einen Ende eine umfangsseitige Verengung auf, so daß der in der Vertiefung 47 abgelegte Motor in der Richtung zur Verengung 50 hin gehalten wird. Insbesondere kann die Verengung 50 so ausgestaltet sein, daß sie eine köcherartige Halterung für den in der Vertiefung 47 abgelegten Antriebsmotor bildet. Zusätzlich kann jedoch auch eine entsprechende Verengung oder köcherartige Halterung auf der zu der Verengung 50 entgegengesetzt liegenden Seite der Vertiefung 47 angeordnet sein.

Gemäß Fig. 6 sind symmetrisch und im wesentlichen parallel zu der Vertiefung 47 zwei schalenförmige Ablagen 48 und 49 angeordnet, die zur Aufnahme eines Behandlungswerkzeugs oder Behandlungsinstrumentes dienen. Gemäß Fig. 6a weisen diese Ablageabschnitte 48 und 49 eine längliche Form auf, wobei diese Ablageabschnitte 48 und 49 jedoch auch an die Form eines zahnärztlichen Handstücks oder Winkelstücks angepaßt sein können. Des weiteren können natürlich auch mehrere dieser Ablageabschnitte 48 und 49 vorgesehen sein. Wie bei der Vertiefung 47 ist auch die Tiefe der schalenförmigen Ablageabschnitte 48 und 49 vorzugsweise so gewählt, daß ein darin abgelegtes Behandlungsinstrument teilweise von der Oberfläche der Ablage 24 hervorsteht, um ein Entnehmen des Behandlungsinstruments zu erleichtern.

Die Seitenwand 46 der in Fig. 6 gezeigten Ablage 24 wird durch einen oberen Rand 44, der durch die Vertiefung 47 unterbrochen wird, sowie einen umlaufenden unteren Rand 45 begrenzt. Wie insbesondere aus Fig. 6b ersichtlich ist, steht der untere Rand aus noch nachfolgend näher zu erläuternden Gründen etwas weiter von der Seitenwand 46 ab. Der obere Rand 44, der untere Rand 45 und die dazwischen angeordnete Seitenwand 46 bestimmen die im wesentlichen ellipsenartige Form der in Fig. 6 gezeigten Ablage 24 und bilden eine Aufnahmeanordnung für den in Fig. 1 gezeigten Versorgungsschlauch 22, da dieser zwischen die beiden Ränder 44 und 45 um die Seitenwand 46 gewickelt werden kann und dabei von den beiden Rändern 44 und 45 gehalten wird. Anstelle der in Fig. 6a dargestellten nahezu vollständig ausgebildeten umlaufenden Form des oberen Randes 44 würde es auch genügen, wenn der obere Rand 44 lediglich teilweise an beispielsweise zwei gegenüberliegenden Stellen der Ablage 24 ausgebildet ist, wodurch ebenfalls bereits eine sichere Halterung des um die Seitenwand 46 gewickelten Versorgungsschlauches gewährleistet wäre.

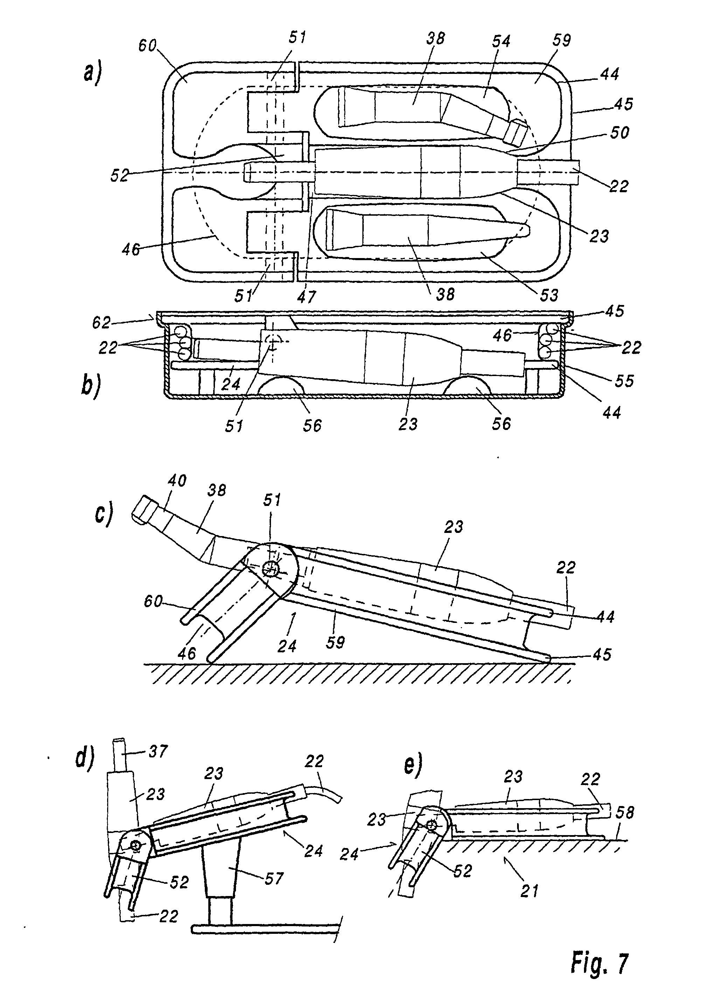

Fig. 7 zeigt in verschiedenen Ansichten ein zweites Ausführungsbeispiel der in Fig. 1 dargestellten Ablage 24, wobei es sich bei diesem Ausführungsbeispiel um eine zweiteilige Ausführungsform handelt. Fig. 7a zeigt eine Draufsicht auf diese Ablage, wobei erkennbar ist, daß zwei Ablageteile 59 und 60 gelenkartig durch entsprechende Gelenkmittel 51 miteinander verbunden sind, so daß insbesondere der Ablageteil 60 gegenüber dem Ablageteil 59 geschwenkt werden kann. Der Ablageteil 59 weist Ablageabschnitte 53 und 54 für die Aufnahme von Behandlungsinstrumente 38 auf, wobei gemäß Fig. 7 beispielhaft hinsichtlich des Ablageabschnitts 54 ein Winkelstück und hinsichtlich des Ablageabschnitts 53 ein Handstück dargestellt ist. Zudem ist in dem Ablageteil 59 die bereits anhand von Fig. 6 beschriebene Vertiefung 47 für den Antriebsmotor 23 mit der Verengung 50 enthalten. Der gegenüber dem Ablageteil 59 schwenk- bzw. drehbare Ablageteil 60 weist eine der Vertiefung 47 im wesentlichen entsprechende Vertiefung 52 auf, wobei diese jedoch in Form einer Köcherhalterung für den Antriebsmotor 23 ausgestaltet ist, so daß der Antriebsmotor 23 einerseits in der Vertiefung 47 abgelegt und andererseits in der köcherartigen Halterung 52 nach Schwenkung des Ablageteils 60 gegenüber dem Ablageteil 59 gehalten werden kann.

Dies ist insbesondere in Fig. 7d und 7e dargestellt, wobei Fig. 7d beispielhaft die Plazierung der Ablage 24 auf einen zu dem in Fig. 1 dargestellten Steuergerät 21 gehörenden Ablagearm 57 darstellt. Fig. 7e zeigt beispielhaft direkt die Auflage der Ablage 24 auf einer Oberfläche 58 des Steuergeräts 21. Sowohl in Fig. 7d als auch in Fig. 7e ist jeweils der Antriebsmotor 23 einerseits in einer in der Vertiefung 47 abgelegten Stellung und andererseits in einer in der Köcherhalterung 52 gehaltenen nahezu senkrechten Stellung dargestellt.

Die zweiteilige Ausführungsform der Ablage 24 weist den Vorteil auf, daß - wie in Fig. 7c gezeigt ist - durch Schwenken des Ablageteils 60 gegenüber dem Ablageteil 59 auf einfache Weise eine gewünschte Neigung der Ablage 24 stufenlos eingestellt werden kann, wobei die Reibung zwischen den in Fig. 7c gezeigten Gelenkmitteln und den beiden Ablageteilen 59 und 60 einerseits so klein ist, daß die Ablageteile 59 und 60 gegeneinander geschwenkt werden können, und andererseits jedoch groß genug ist, daß auch nach Ablage des Antriebsmotors 23 sowie der Behandlungsinstrumente 38 in der Ablage 24 die gemäß Fig. 7c herbeigeführte Stellung der Ablage 24 beibehalten werden kann.

Wie bei dem in Fig. 6 gezeigten Ausführungsbeispiel steht auch bei dem in Fig. 7 dargestellten Ausführungsbeispiel der untere Rand 45 weiter als der obere Rand 44 von der Seitenwand 46 seitlich ab. Dadurch kann die Ablage 24 in eine in Fig. 7b gezeigte Sterilisationskassette 55 eingesetzt werden, wobei die Ablage 24 in der Sterilisationskassette 55 mit dem Rand 44 nach unten gerichtet abgelegt ist. Ein in der Ablage gehaltener Antriebsmotor 23 kommt dabei auf entsprechenden Auflageflächen 56 zu liegen. Der weiter von der Seitenwand 46 der Auflage 24 hervorstehende Rand 45 liegt dabei auf Schultern 62 der Sterilisationskassette 55 auf, so daß die Ablage 24 zugleich als "Verpackung" für die in der Ablage 24 gehaltenen Instrumente bzw. den Antriebsmotor 23 und den Versorgungsschlauch 22 dient. Aus Fig. 7b ist insbesondere auch der auf die Seitenwand 46 der Ablage 24 aufgewickelte Versorgungsschlauch 22 ersichtlich.

Bei der in Fig. 7b dargestellten Sterilisationskassette 55 handelt es sich um einen mehrfach sterilisierbaren Behälter, in den durch entsprechend vorgesehene Öffnungen Sterilisierdampf bzw. Sterilisiergas zur Sterilisierung der Ablage 24 und der darin gehaltenen Instrumente, des Antriebsmotors 23 und des Versorgungsschlauches 22 eingeführt werden kann. Eine derartige Sterilisationskassette ist beispielsweise aus der EP-A1-0588 228 oder der DE-U1-92 06 022 bekannt. In der Regel erfolgt die Sterilisation bei einer Temperatur von bis zu 135° C. Während beim bekannten Stand der Technik die einzelnen zu sterilisierenden Teile in einen Klarsichtbeutel verpackt werden müssen, können die in der in Fig. 6 und 7 gezeigten Ablage 24 abgelegten Teile zusammen mit der Ablage 24 in einer entsprechenden Sterilisationskassette sterilisiert werden, ohne daß eine Verpackung in Klarsichtbeutel erforderlich ist. Zudem können die einzelnen Behandlungsinstrumente bequem zusammen mit dem Antriebsmotor und dem Versorgungsschlauch sterilisiert werden. Allgemein ist die Ablage 24 derart geformt, daß sie in eine übliche Sterilisationskassette, Sterilisationstray oder einen üblichen Sterilisationskorb vorzugsweise schlüssig eingesetzt werden kann.

Nachdem die zu sterilisierenden Teile zusammen mit der Ablage 24 sterilisiert werden, muß die Ablage 24 aus einem sterilisierbaren Material, wie z.B. Silikon, hergestellt sein. Die Ablage 24 ist frei positionierbar und kann durch Befestigungsmittel an entsprechenden Stellen des in Fig. 1 gezeigten Steuergerätes 21 oder - wie in Fig. 7d angedeutet - auf einem Ablagearm 57 des Steuergeräts befestigt werden.

Insbesondere bei Anwendungen in der Implantologie sind zum Teil sehr genaue Drehmomente erforderlich. Diese Drehmomente sind nicht allein vom Strom des Antriebsmotors abhängig, sondern auch vom Zustand des mit dem Antriebsmotor gekoppelten Behandlungsinstrumentes. Es wird daher vorgeschlagen, für eine Drehmomentgenauigkeit von kleiner ±3% den Antriebsmotor mit darauf aufgesetztem Behandlungsinstrument zu kalibrieren. Bei der Drehmomentkalibrierung wird das tatsächlich von dem Antriebsmotor und dem damit gekoppelten Behandlungsinstrument ausgeübte Drehmoment bzw. die entsprechende Drehmomentkennlinie ermittelt und anschließend der Antriebsmotor mit dem daran gekoppelten Behandlungsinstrument gemäß dem ermittelten Drehmoment bzw. der ermittelten Drehmomentkennlinie angesteuert, so daß der Betrieb des Antriebsmotors und des Behandlungsinstruments durch die Drehmomentkalibrierung auf das tatsächlich von dem Antiebsmotor und dem Behandlungsinstrument ausgeübte Drehmoment abgestimmt ist.

Die Drehmomentkalibrierung bzw. die Ermittlung des Drehmoments erfolgt mit Hilfe einer Drehmomentermittlungseinrichtung, die einerseits in das in Fig. 1 gezeigte Steuergerät 21 integriert oder andererseits als eine an das Steuergerät 21 anschließbare separate Einheit vorgesehen sein kann. Vorteilhafterweise umfaßt die Drehmomentermittlungseinrichtung ein Softwaremodul mit Bedienerführung, mit dessen Hilfe auf einer der Drehmomentermittlungseinrichtung zugeordneten Anzeigeneinheit oder auf der Anzeigeneinheit 36 des Steuergeräts 21 entsprechende Ablauf- bzw. Bedieninformationen für die Durchführung der Drehmomentkalibrierung angezeigt werden.

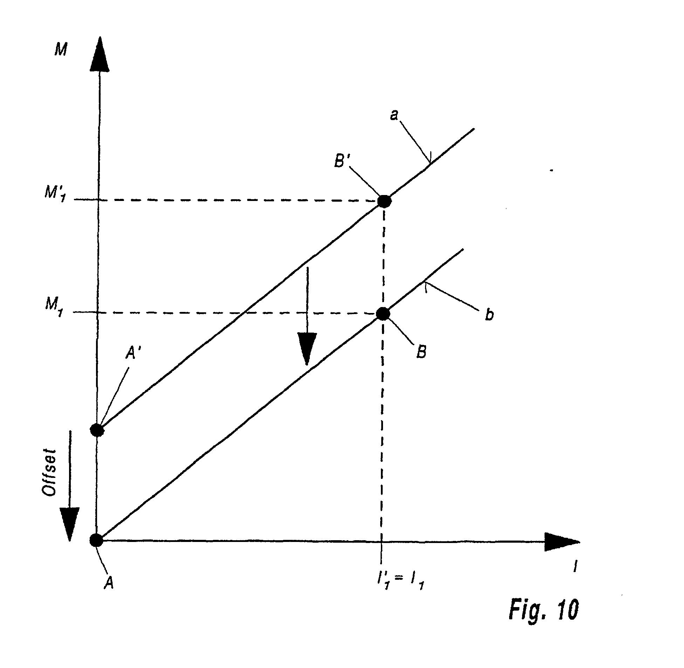

Zur Durchführung der Drehmomentkalibrierung muß zunächst im Falle einer externen Drehmomentermittlungseinrichtung diese Drehmomentermittlungseinrichtung an das Steuergerät 21 angeschlossen und anschließend - wie bereits anhand von Fig. 2 erläutert worden ist - die in Fig. 2 gezeigte Taste 12 betätigt werden, um die Drehmomentkalibrierungsprozedur zu starten. Anschließend wird mit Hilfe der zuvor beschriebenen Bedienerführung der Benutzer zur Durchführung der nachfolgend näher anhand von Fig. 10 beschriebenen Drehmomentkalibrierungsprozedur aufgefordert.

Fig. 10 zeigt zwei Drehmoment-Motorstrom-Kennlinen a und b. Die Kennlinie a zeigt die sich bei Kopplung eines Behandlungsinstruments mit dem Antriebsmotor ergebende Kennlinie ohne Drehmomentkalibrierung. Mit Hilfe der Drehmomentkalibrierung soll nunmehr die Ist-Kennlinie a zu der Soll-Kennlinie b verschoben werden. Die Soll-Kennlinie b entspricht derjenigen Kennlinie, mit der das in Fig. 1 und 2 gezeigte Steuergerät 21 normalerweise den Antriebsmotor mit dem daran gekoppelten Behandlungsinstrument ansteuern würde. Die Soll-Kennlinie b ist demnach dem Steuergerät 21 bekannt. Zur Eliminierung des in Fig. 10 gezeigten Offset zwischen den Kennlinien a und b genügt es somit, einen bestimmten Meßpunkt B' der Ist-Kennlinie a zu ermitteln, so daß aufgrund des Unterschieds zwischen dem dem Meßpunkt B' zugeordneten Drehmoment M'₁ und dem dem Soll-Meßpunkt B' zugeordneten Drehmoment M₁ auf die Verschiebung zwischen den beiden Kennlinien a und b geschlossen werden kann, um diese anschließend zu eliminieren. Bei der Eliminierung dieses Offset wird die Ist-Kennlinie a zu der Soll-Kennlinie b verschoben, so daß der im Leerlauf des Antriebsmotors (I = 0) auftretende Drehmomentwert A' zum Meßpunkt A verschoben und somit als neuer Nullpunkt behandelt wird.

Zur Ermittlung des in Fig. 10 dargestellten Meßpunktes B' muß zum einen der entsprechende Drehmomentwert M'₁ und zum anderen der Motorstromwert I'₁ ermittelt werden. Der Motorstrom kann beispielsweise wie in der DE-A1-196 28 854 mit Hilfe eines in Serie mit dem Antriebsmotor geschalteten Widerstands erfaßt werden, wobei die an dem Widerstand abfallende Spannung als proportionale Größe zu dem Motorstrom ausgewertet wird.

Anhand Fig. 10 wurde der Fall erläutert, daß die Ist-Kennlinie a parallel zu der Soll-Kennlinie b verläuft. Unter bestimmten Umständen kann jedoch die Ist-Kennlinie a auch geneigt zu der Soll-Kennlinie b verlaufen. In diesem Fall müssen zur Ermittlung der Ist-Kennlinie a zwei Meßpunkte ermittelt werden, die die Ist-Kennlinie a eindeutig definieren. Vorteilhafterweise ist eine der beiden Meßpunkte der Meßpunkt A' im Leerlauf des Antriebsmotors (I = 0), so daß aufgrund des dem Meßpunkt A' entsprechenden Drehmomentwertes die Ist-Kennlinie in den Nullpunkt verschoben werden kann.

Allgemein kann z.B. vorgesehen sein, daß die Drehmomentermittlungseinrichtung den Motorstrom bzw. die Motorleistung standardmäßig für z.B. drei verschiedene Drehmomentwerte, wie z.B. 0 Ncm (Leerlauf), 5 Ncm und 35 Ncm, mißt, um daraus die für die Kalibrierung erforderlichen Drehmomentdaten zu gewinnen. Liegt ein Meßwert außerhalb vorgegebener Meßwertgrenzen, kann die Bedienperson über die Anzeige 36 zur Überprüfung des Antriebsmotors 23 bzw. des darauf aufgesetzten Behandlungsinstrumentes 38 oder zur Wiederholung der Drehmomentkalibrierung aufgefordert werden.

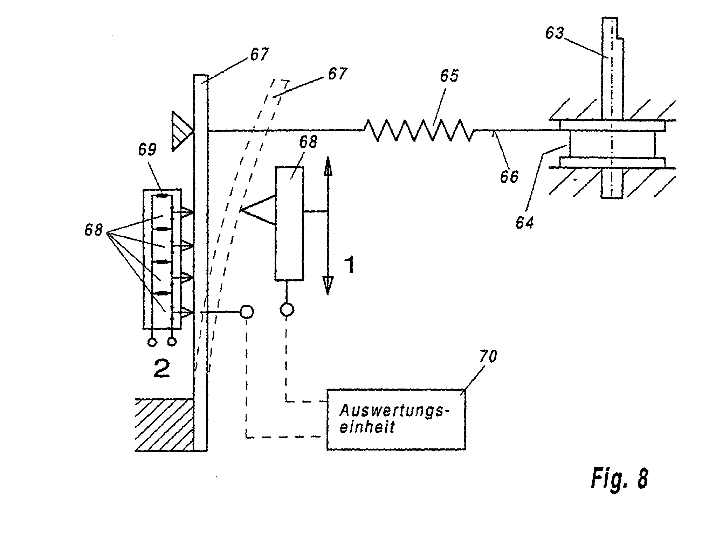

Fig. 8 zeigt ein erstes Ausführungsbeispiel einer Drehmomentermittlungseinrichtung, mit der das tatsächlich von dem Antriebsmotor und dem darauf aufgesetzten Behandlungsinstrument ausgeübte Drehmoment ermittelt werden kann. Gemäß Fig. 8 wird das von dem Antriebsmotor sowie dem Behandlungsinstrument ausgeübte Drehmoment in eine Verstellkraft für ein Verstellmedium umgesetzt, wobei der von der Verstellkraft hervorgerufene Verstellweg des Verstellmediums erfaßt und in ein entsprechendes elektrisches Signal umgewandelt wird. Bei dem in Fig. 8 gezeigten Ausführungsbeispiel ist das Verstellmedium eine einseitig eingespannte und vorgespannte Blattfeder 67. Das von dem Antriebsmotor und dem Behandlungsinstrument ausgeübte Drehmoment wird von einem Mitnehmer bzw. einer Aufnahmewelle 63 aufgenommen, welche mechanisch mit einer Wickeltrommel 64 gekoppelt ist. Die Wickeltrommel 64 ist über ein Spannseil 66 mit dem nicht eingespannten Ende der Blattfeder 67 verbunden. In das Spannseil ist eine Feder 65 zur Dämpfung und Verringerung des Spannweges eingesetzt, die zudem eine Kraftvorspannung des Seils ermöglicht.

Bei Ausübung eines bestimmten Drehmoments des Antriebsmotors mit dem daran gekoppelten Behandlungsinstrument wird die Aufnahmewelle 63 gedreht und das Seil 66 entsprechend auf der Wickeltrommel 64 aufgewickelt. Bei einer bestimmten, geeichten Kraft verläßt die Blattfeder 67 ihre ursprüngliche Position und wird in die in Fig. 8 gestrichelt gezeichnete Auslenkposition bewegt. Diese Auslenkung bzw. dieser Verstellweg ist ein Maß für das auf die Aufnahmewelle 63 ausgeübte Drehmoment. Sobald an der Aufnahmewelle 63 kein Drehmoment mehr anliegt, bewegt sich die Blattfeder 67 aufgrund ihrer Vorspannung zurück in ihre Ausgangsposition und das Seil 66 wird wieder von der Wickeltrommel 64 abgewickelt.

Zur Erfassung des Verstellweges, d.h. der Auslenkung, der Blattfeder 67 ist eine Schaltanordnung 68 vorgesehen, wobei gemäß Fig. 8 zwischen zwei Varianten 1 und 2 unterschieden wird. Gemäß der Variante 1 ist ein Schalter 68 vorgesehen, der zusammen mit der Blattfeder 67 sowie einer Auswertungseinheit 70 einen Stromkreis bildet. Bei einer bestimmten geeichten Verstellkraft des Seils 66 wird der Schalter 68 betätigt und dadurch eine Signaländerung ausgelöst, die der Auswertungseinheit 70 zugeführt und mitgeteilt wird. Der Schalter 68 kann fest oder aber auch insbesondere entsprechend den Meßwerten in Pfeilrichtung verschiebbar angeordnt werden. Durch Verschieben des Schalters 68 nach oben kann demnach ein kleineres Drehmoment und durch Verschieben des Schalters 68 nach unten ein größeres Drehmoment erfaßt werden. Die Auswertungseinheit 70 setzt das von dem Schalter 68 gelieferte Signal bzw. die entsprechende Signaländerung mit Hilfe einer entsprechenden Software in das zu messende Drehmoment um. Mit Hilfe des von der Auswertungseinheit 70 gelieferten Drehmoments ist somit eine Korrektur der in Fig. 10 gezeigten Ist-Kennlinie a möglich, und der Antriebsmotor mit dem daran gekoppelten Behandlungsinstrument kann mit größtmöglicher Genauigkeit unter Berücksichtigung des in Fig. 10 dargestellten Offset betrieben werden. Die in Fig. 8 gezeigte Auswertungseinheit 70 kann in der Drehmomentermittlungseinrichtung oder aber auch in dem in Fig. 1 und 2 gezeigten Steuergerät 21 der ärztlichen Behandlungsvorrichtung enthalten sein.

Gemäß der durch 2 in Fig. 8 bezeichneten zweiten Variante der Schalteranordnung 68 sind mehrere einzelne Schalter in Längsrichtung der Blattfeder 67 vorgesehen, die jeweils mit entsprechenden Widerständen 69 gekoppelt sind und somit ein Schalter-Widerstandsnetzwerk bilden. Abhängig von der Auslenkung der Blattfeder 67 werden somit unterschiedliche Schalter dieser Schalteranordnung betätigt, so daß an den Ausgangsanschlüssen dieser Schalteranordnung abhängig von der Auslenkung der Blattfeder 67 ein unterschiedlicher Gesamt-Widerstandswert auftritt, der von der Auswertungseinheit 70 ausgewertet und in das entsprechende Drehmoment umgerechnet werden kann.

Anstelle der in Fig. 8 gezeigten Schalteranordnung kann beispielsweise auch ein Potentiometer verwendet werden, dessen Schleifkontakt mit der Blattfeder 67 mechanisch gekoppelt ist, so daß sich das von dem Potentiometer gelieferte Ausgangssignal proportional mit der Auslenkung der Blattfeder 67 verändert. Das Ausgangssignal des Potentiometers wird dabei ebenfalls der in Fig. 8 gezeigten Auswertungseinheit 70 zugeführt.

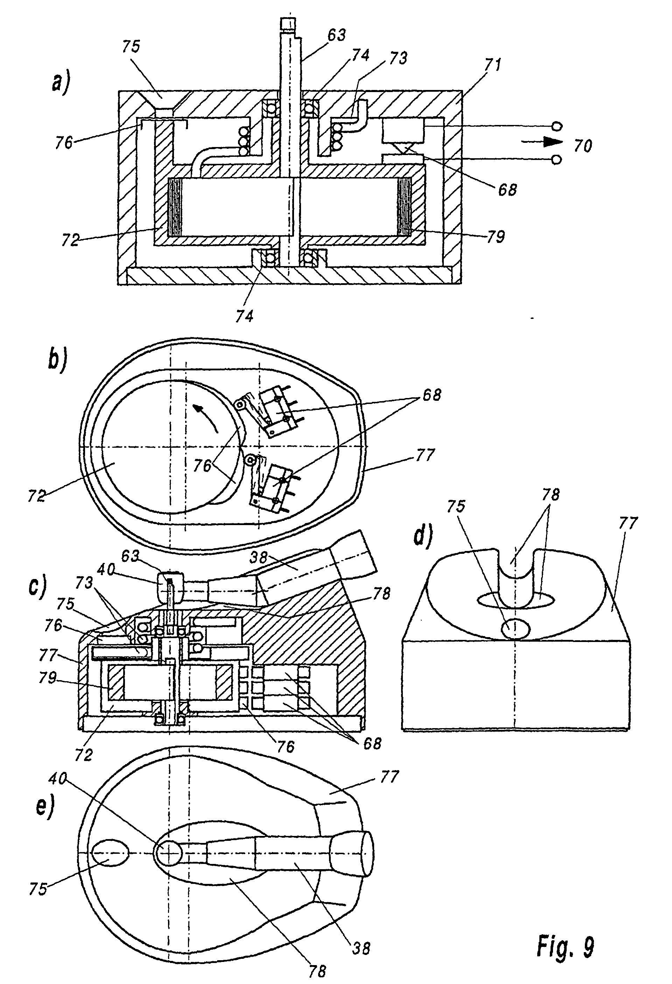

Fig. 9 zeigt verschiedene Ansichten eines weiteren Ausführungsbeispiels der zuvor beschriebenen Drehmomentermittlungseinrichtung, welche im Sinne einer Hysteresebremse realisiert ist. Fig. 9a zeigt eine Querschnittsansicht dieser Einrichtung, wobei ebenfalls eine Aufnahmewelle 63 zur Aufnahme bzw. Übertragung des von dem Antriebsmotor mit dem Behandlungsinstrument ausgeübten Drehmoments vorgesehen ist. Die Welle 63 ist mit einem durch Kugellager 74 in einem Außengehäuse 71 drehbar gelagerten Gehäuse- bzw. Drehkörper 72 verbunden, wobei dieser Drehkörper 72 über eine Spiralfeder 73 mit dem Außengehäuse 71 gekoppelt ist. Bei Ausübung eines Drehmoments auf den Mitnehmer bzw. die Aufnahmewelle 63 wird dieser Drehkörper 72 in dem Außengehäuse 71 verdreht, und es werden analog zu dem in Fig. 8 gezeigten Ausführungsbeispiel abhängig von dem Verdrehwinkel entsprechend angeordnete Schaltermittel 68 aktiviert, deren Ausgangssignale der in Fig. 8 gezeigten Auswertungseinheit 70 zugeführt werden, die abhängig von den Signalveränderungen der Schaltermittel das entsprechende Drehmoment bestimmt. In dem Drehkörper 72 ist eine Flachspiralfeder 79 mit beispielsweise 20 oder 40 Wicklungen vorhanden, die sich bei Verdrehen der Aufnahmewelle 63 auf der Aufnahmewelle 63 abwickelt und somit eine Rückstellkraft für den Drehkörper 72 ausübt, um diesen wieder in die ursprüngliche Position zurückzubewegen, wenn kein Drehmoment mehr auf die Aufnahmewelle 63 ausgeübt wird. Das Außengehäuse 71 besitz ein Fenster 75, so daß durch dieses Fenster eine Ableseskala bzw. Analoganzeige 76 des Drehkörpers 72 beobachtet werden kann. Vorzugsweise weist die Analoganzeige 76 direkt die der Verdrehung des Drehkörpers 72 entsprechenden Drehmomentwerte auf, so daß der Benutzer unmittelbar abhängig von der Verdrehung des Drehkörpers 72 das entsprechende Drehmoment ablesen kann.

Fig. 9b - 9e zeigen verschiedene Ansichten einer Variante des in Fig. 9a gezeigten Ausführungsbeispiels, wobei Fig. 9c die entsprechende Querschnittsansicht, Fig. 9d eine Frontalansicht und Fig. 9e eine Draufsicht dieser Variante darstellt. Fig. 9b zeigt eine Draufsicht auf den Innenraum der in Fig. 9c dargestellten Variante mit den wesentlichen Bauteilen.

Die in Fig. 9c dargestellten Elemente entsprechen im wesentlichen den in Fig. 9a dargestellten Elementen und sind mit entsprechenden Bezugszeichen versehen. Die Drehmomentermittlungseinrichtung ist jedoch anstelle des in Fig. 9a gezeigten Gehäuses in einen Gehäusekörper 77 eingebaut, der zudem eine Ausnehmung bzw. eine Ablagenut 78 für ein Behandlungsinstrument, gemäß Fig. 9c ein Winkelstück 38 mit daran gekoppeltem Kopfteil 40 aufweist, wobei das Behandlungsinstrument 38 in der Ausnehmung 78 abgelegt und zugleich in dieser Ablagestellung mechanisch mit der Aufnahmewelle 63 der Drehmomentermittlungseinrichtung gekoppelt werden kann. Aus Fig. 9c in Verbindung mit Fig. 9b ist zudem ersichtlich, daß die Außenseite des Drehkörpers 72 mehrere hervorstehende Schaltnocken 76 aufweist, die bei Drehung des Drehkörpers 72 in der in Fig. 9b dargestellten Pfeilrichtung entsprechend angeordnete Schalter abhängig von dem Verdrehwinkel des Drehkörpers 72 betätigen. Wie in Fig. 9c gezeigt ist, können mehrere dieser Schaltnocken 76 sowie die entsprechenden Schalter 68 vertikal übereinanderliegend angeordnet sein. Vorzugsweise sind die Schaltnocken mit der in Fig. 9b gezeigten Form gleichmäßig entlang des Umfangs des Drehkörpers 72 verteilt. Selbstverständlich können die Schaltnocken auch eine andere Außenform besitzen, soweit dadurch eine Betätigung der Schalter 68 möglich ist. Die Ausgangssignale der Schalter 68 werden wiederum der in Fig. 8 gezeigten Auswertungseinheit zugeführt, die abhängig von den von den Schaltern 68 gelieferten Signalveränderungen auf das auf die Aufnahmewelle 63 ausgeübte Drehmoment des Antriebsmotors mit dem daran gekoppelten Behandlungsinstrument schließt.

Zur Vorgabe bestimmter Drehmomentwerte können für die Aufnahmewelle 63 bzw. den Drehkörper 72 entsprechende Raststufen vorgesehen sein, die durch in Umfangsrichtung der Aufnahmewelle 63 bzw. des Drehkörpers 72 angeordnete (nicht gezeigte) Kugelraststifte realisiert sein können. Jede Raststufe entspricht dabei einem bestimmten Drehmoment, wie z.B. 0 Ncm, 15 Ncm oder 30 Ncm. Durch Einrasten der Aufnahmewelle 63 mit dem aufgesetzten Behandlungswerkzeug 38 bzw. des Drehkörpers 72 in die entsprechenden Raststufen kann der dem jeweiligen vorgegebenen Drehmoment entsprechende Motorstrom gemessen werden, um somit die gewüschten Drehmomentkennliniendaten gewinnen zu können.

Bei jedem der in Fig. 8 und 9 gezeigten Ausführungsbeispiele liefert die Auswertungseinheit 70 der entsprechenden Drehmomentermittlungseinrichtung das tatsächlich von dem Behandlungsinstrument und dem Antriebsmotor auf die Aufnahmewelle 63 ausgeübte Drehmoment bzw. ein diesem Drehmoment entsprechendes Signal, welches dem Steuergerät 21 bzw. einer in dem Steuergerät 21 vorhandenen Steuereinheit zugeführt wird, so daß das Steuergerät abhängig von dem somit ermittelten Drehmomentwert die Drehmomentkalibrierung, d.h. die anhand von Fig. 10 erläuterte Drehmomentkorrektur, durchführen und das Behandlungsinstrument mit größtmöglicher Genauigkeit ansteuern kann, indem der Antriebsmotor 23 mit einer entsprechenden Leistungszufuhr betrieben wird.

Bei den zuvor beschriebenen Drehmomentermittlungseinrichtungen wurde davon ausgegangen, daß das von einem auf den Antriebsmotor 23 aufgesetzten Behandlungsinstrument 38 ausgeübte Drehmoment ermittelt wird (z.B. mit Hilfe der Aufnahmewelle 63). Alternativ kann selbstverständlich auch eine Abwandlung dahingehend erfolgen, daß das Behandlungsinstrument 38 mit einem Behandlungswerkzeug 39 (z.B. einem Bohrer) gekoppelt ist und das von dem auf das Behandlungsinstrument 38 aufgesetzten Bohrer 39 ausgeübte Drehmoment ermittelt und zur Grundlage der Kalibrierung gemacht wird. Zudem besteht die Möglichkeit, daß die Drehmomentermittlungseinrichtung direkt, d.h. ohne die Hilfe des Steuergeräts 21, abhängig von den ermittelten Drehmomentdaten die Leistungszufuhr des Antriebsmotors 23 entsprechend einstellt.

Abschließend sei erwähnt, daß das in Fig. 1 und 2 gezeigte Steuergerät 21 der ärztlichen Behandlungsvorrichtung 20 auch einen Druckeranschluß aufweisen kann, so daß verschiedene technische Informationen, wie z.B. die auf der Anzeigeneinheit 36 dargestellten Betriebsparameterinformationen, das Datum, die Uhrzeit usw. ausgedruckt werden können. Anhand eines derartigen Ausdrucks kann anschließend der Behandlungsablauf nachvollzogen und beurteilt werden. Ebenso kann eine serielle oder parallele Schnittstelle zum Anschluß des Steuergeräts an einen Computer vorgesehen sein, um Daten an den Computer übertragen zu können und am Bildschirm des Computers eine vergrößerte Displayanzeige darstellen bzw. mit Hilfe des Computers Daten aufzeichnen zu können.

## Patentansprüche

1. Ärztliche Behandlungsvorrichtung (20),
mit einem Steuergerät (21),
mit einer an das Steuergerät (21) anschließbaren Versorgungsleitung (22), über die das Steuergerät (21) den Betrieb eines Behandlungswerkzeugs (39) steuert, und
mit Auswahlmitteln (1, 2), um zwischen einer Vielzahl von fest vorgegebenen, von einem Benutzer nicht veränderbaren ersten Betriebsprogrammen einerseits und einer Vielzahl von von dem Benutzer beliebig veränderbaren zweiten Betriebsprogrammen andererseits zu wählen,
wobei das Steuergerät (21) den Betrieb des Behandlungswerkzeugs (39) abhängig von durch ein ausgewähltes erstes oder zweites Betriebsprogramm bereitgestellten Betriebsparameterinformationen steuert.

2. Ärztliche Behandlungsvorrichtung nach Anspruch 1,
**dadurch gekennzeichnet,**
**daß** an die Versorgungsleitung (22) ein Antriebsmotor (23) angeschlossen ist, mit dem ein Behandlungsinstrument (38, 40) koppelbar ist, wobei das Behandlungswerkzeug (39) wiederum mit dem Behandlungsinstrument (38, 40) koppelbar ist.

3. Ärztliche Behandlungsvorrichtung nach einem der vorhergehenden Ansprüche,
**dadurch gekennzeichnet,**
**daß** mit Hilfe der Auswahlmittel (1, 2, 3) zudem ein Einstellmodus auswählbar ist, in dem bestimmte Betriebsparameter durch den Benutzer einstellbar sind.

4. Ärztliche Behandlungsvorrichtung nach Anspruch 2,
**dadurch gekennzeichnet,**
**daß** in dem Steuergerät (21) eine Datenbank (41, 42) gespeichert ist, welche Informationen über die verfügbaren Behandlungsinstrumente (38, 40) und/oder die verfügbaren Behandlungswerkzeuge (39) umfaßt.

5. Ärztliche Behandlungsvorrichtung nach Anspruch 4,
**dadurch gekennzeichnet,**
**daß** die Datenbank (41) für jedes verfügbare Behandlungsinstrument (38, 40) Informationen über die Drehzahlübertragung, den Wirkungsgrad, das maximale Drehmoment, die maximale Antriebsdrehzahl und/oder den beabsichtigten Betriebsdrehzahlbereich umfaßt.

6. Ärztliche Behandlungsvorrichtung nach Anspruch 3 und Anspruch 4 oder 5,
**gekennzeichnet durch**
Einstellmittel (6 - 9), um in dem Einstellmodus anhand der in der Datenbank (41, 42) abgelegten Informationen ein gewünschtes Behandlungsinstrument (38, 40) und/oder ein gewünschtes Behandlungswerkzeug (39) einzustellen.

7. Ärztliche Behandlungsvorrichtung nach einem der vorhergehenden Ansprüche,
**gekennzeichnet durch**
eine Kühlmittelpumpeinrichtung (31, 61), um dem Behandlungswerkzeug (39) ein Kühlmittel zuzuführen.

8. Ärztliche Behandlungsvorrichtung nach den Ansprüchen 6 und 7,
**dadurch gekennzeichnet,**
**daß** die Datenbank (41 - 43) auch Informationen über mögliche Pumpleistungen oder mögliche Kühlmittelmengen der Kühlmittelpumpeinrichtung (31, 61) umfaßt, und
**daß** mit Hilfe der Einstellmittel (6 - 9, 13) anhand der in der Datenbank (41 - 43) abgelegten Informationen eine gewünschte Pumpleistung oder Kühlmittelmenge der Kühlmittelpumpeinrichtung (31, 61) eingestellt werden kann.

9. Ärztliche Behandlungsvorrichtung nach Anspruch 6 oder 8,
**gekennzeichnet durch**
Speichermittel (5), um nach dem Einstellen der bestimmten Betriebsparameter mit Hilfe der Einstellmittel (6 - 9, 13) die eingestellten Betriebsparameter als ein zweites Betriebsprogramm zu speichern.

10. Ärztliche Behandlungsvorrichtung nach Anspruch 2,
**dadurch gekennzeichnet,**
**daß** die einem ersten oder zweiten Betriebsprogramm entsprechenden Betriebsparameterinformationen den Typ eines bestimmten Behandlungsinstruments (38, 40), die Drehzahlübertragung, den Wirkungsgrad, das maximale Drehmoment, die maximale Antriebsdrehzahl und/oder den beabsichtigten Betriebsdrehzahlbereich des bestimmten Behandlungsinstruments (38, 40) sowie den Typ eines bestimmten Behandlungswerkzeugs (39) umfassen.

11. Ärztliche Behandlungsvorrichtung nach einem der vorhergehenden Ansprüche,
**gekennzeichnet durch**
eine Anzeigeneinheit (36), um nach Auswahl eines ersten oder zweiten Betriebsprogramms die dem ausgewählten Betriebsprogramm entsprechenden Betriebsparameterinformationen anzuzeigen.

12. Ärtzliche Behandlungsvorrichtung nach Anspruch 11 und einem der Ansprüche 6, 8 oder 9,
**dadurch gekennzeichnet,**
**daß** die Anzeigeneinheit (36) auch das mit Hilfe der Einstellmittel (6 - 9) eingestellte Behandlungsinstrument (38, 40) bzw. Behandlungswerkzeug (39) anzeigt.

13. Ärztliche Behandlungsvorrichtung nach Anspruch 11 oder 12,
**dadurch gekennzeichnet,**
**daß** die Anzeigeneinheit (36) auch Fehlermeldungen und/oder Hilfeinformationen anzeigt.

14. Ärztliche Behandlungsvorrichtung nach Anspruch 2,
**dadurch gekennzeichnet,**
**daß** der Antriebsmotor (23) ein kollektorloser Motor mit einem Antriebsdrehzahlbereich von 200 min⁻¹ - 40 000 min⁻¹ ist.

15. Ärztliche Behandlungsvorrichtung nach Anspruch 2 oder 14,
**dadurch gekennzeichnet,**
**daß** an das Steuergerät (21) ein zweiter Antriebsmotor (21) angeschlossen ist.

16. Ärztliche Behandlungsvorrichtung nach Ansprüche 7 und 15,
**gekennzeichnet durch**
eine zweite Kühlmittelpumpeinrichtung (31, 61), um einem von dem zweiten Antriebsmotor (23) betriebenen Behandlungswerkzeug (39) ein Kühlmittel zuzuführen.

17. Ärztliche Behandlungsvorrichtung nach einem der vorhergehenden Ansprüche,
**gekennzeichnet durch**
eine Pneumatik-Antriebseinrichtung (33), um ein daran anschließbares Behandlungswerkzeug (34) pneumatisch zu betreiben.

18. Ärztliche Behandlungsvorrichtung nach Anspruch 17,
**dadurch gekennzeichnet,**
**daß** der von der Pneumatik-Antriebseinrichtung (33) gelieferte Antriebsluftdruck einstellbar ist.

19. Ärztliche Behandlungsvorrichtung nach einem der vorhergehenden Ansprüche,
**gekennzeichnet durch**
eine Schnittstelle zum Anschluß eines Druckers, um bestimmte Betriebsinformationen der ärztlichen Behandlungsvorrichtung auf dem Drucker auszudrucken.

20. Ärztliche Behandlungsvorrichtung nach einem der vorhergehenden Ansprüche,
**gekennzeichnet durch**
einen kabellosen Fußschalter (25), der Steuersignale an das Steuergerät (21) zur Ansteuerung des Steuergeräts überträgt.

21. Ärztliche Behandlungsvorrichtung nach Anspruch 20,
**dadurch gekennzeichnet,**
**daß** mit Hilfe des Fußschalters (25) ein gewünschtes Betriebsprogramm aus den ersten und zweiten Betriebsprogrammen auswählbar ist.

22. Ärztliche Behandlungsvorrichtung nach Anspruch 20 oder 21,
**dadurch gekennzeichnet,**
**daß** der Fußschalter (25) mit einem Akkumulator oder einer Batterie betrieben ist.

23. Ärztliche Behandlungsvorrichtung nach Anspruch 20 und Anspruch 2 oder 15,
**dadurch gekennzeichnet,**
**daß** mit Hilfe des Fußschalters (25) die Antriebsdrehzahl und die Drehrichtung des Antriebsmotors (23) veränderbar ist.

## Claims

1. Medical treatment apparatus (20),
having a control device (21),
having a supply line (22) connectable to the control device (21), via which supply line the control device (21) controls the operation of a treatment tool (39), and
having selection means (1, 2) for selection on the one hand between a plurality of fixedly predetermined first operating programs, not alterable by a user, and
on the other hand between a plurality of second operating programs, which can be changed as desired by the user,
the control device (21) controlling the operation of the treatment tool (39) in dependence upon operating parameter information made available by a selected first or second operating program.

2. Medical treatment apparatus according to claim 1,
**characterized in that**,
a drive motor (23) is connected to the supply line (22), to which motor a treatment instrument (38, 40) can be coupled, the treatment tool (39) being couplable in turn with the treatment instrument (38, 40).

3. Medical treatment apparatus according to any preceding claim,
**characterized in that**,
with the aid of the selection means (1, 2, 3) a setting mode can also be selected in which certain operating parameters can be set by the user.

4. Medical treatment apparatus according to claim 2,
**characterized in that**,
there is stored in the control device (21) a data bank (41, 42) which includes information about the available treatment instruments (38, 40) and/or the available treatment tools (39).

5. Medical treatment apparatus according to claim 4,
**characterized in that**,
the data bank (41) includes for each available treatment instrument (38, 40) information about the rotary speed transmission, the efficiency, the maximum torque, the maximum drive rotary speed and/or the intended operational rotary speed range.

6. Medical treatment apparatus according to claim 3 and claim 4 or 5,
**characterized by**
setting means (6-9) in order, in the setting mode, to set up a desired treatment instrument (38, 40) and/or a desired treatment tool (39) on the basis of the information stored in the data bank (41, 42).

7. Medical treatment apparatus according to any preceding claim,
**characterized by**
a coolant pump arrangement (31, 61) for delivering a coolant to the treatment tool (39).

8. Medical treatment apparatus according to claim 6 and 7,
**characterized in that**,
the data bank (41-43) also includes information about possible pump powers or possible coolant quantities of the coolant pump arrangement (31, 61), and
**in that** with the aid of the setting means (6-9, 13) a desired pump power or coolant quantity of the coolant pump arrangement (31, 61) can be set on the basis of the information stored in the data bank (41-43).

9. Medical treatment apparatus according to claim 6 or 8,
**characterized by**
storage means (5) for storing, after the setting of the predetermined operating parameters with the aid of the setting means (6-9, 13), the set operating parameters as a second operating program.

10. Medical treatment apparatus according to claim 2,
**characterized in that**,
the operating parameter information corresponding to a first or second operating program includes the type of a certain treatment instrument (38, 40), the rotary speed transmission, the efficiency, the maximum torque, the maximum rotary drive speed and/or the intended operational rotary speed range of the certain treatment instrument (38, 40), as well as the type of a particular treatment tool (39).

11. Medical treatment apparatus according to any preceding claim,
**characterized by**
a display unit (36) in order, after selection of a first or second operating program, to display the operating parameter information corresponding to the selected operating program.

12. Medical treatment apparatus according to claim 11 and any of claims 6, 8 or 9,
**characterized in that**,
the display unit (36) also shows the treatment instrument (38, 40) or treatment tool (39) set with the aid of the setting means (6-9).

13. Medical treatment apparatus according to claim 11 or 12,
**characterized in that**,
the display unit (36) also displays fault reports and/or help information.

14. Medical treatment apparatus according to claim 2,
**characterized in that**,
the drive motor (23) is a collectorless motor having a drive rotary speed range from 200 min⁻¹ - 40000 min⁻¹.

15. Medical treatment apparatus according to claim 2 or 14,
**characterized in that**,
there is connected to the control device (21) a second drive motor (21).

16. Medical treatment apparatus according to claim 7 and 15,
**characterized by**
a second coolant pump arrangement (31, 61) in order to deliver a coolant to a treatment tool (39) driven by the second drive motor (23).

17. Medical treatment arrangement according to any preceding claim,
**characterized by**
a pneumatic drive arrangement (33), in order to pneumatically drive a treatment tool (34) connectable thereto.

18. Medical treatment apparatus according to claim 17,
**characterized in that**,
the drive air pressure delivered by the pneumatic drive arrangement (33) is settable.

19. Medical treatment apparatus according to any preceding claim,
**characterized by**
an interface for the connection of a printer, in order to print out on the printer certain operational information of the medical treatment apparatus.

20. Medical treatment apparatus according to any preceding claim,
**characterized by**
a cableless foot switch (25) which transmits control signals to the control device (21) for the control of the control device.

21. Medical treatment apparatus according to claim 20,
**characterized in that**,
with the aid of the foot switch (25) a desired operational program can be selected from the first and second operation programs.

22. Medical treatment apparatus according to claim 20 or 21,
**characterized in that**,
the foot switch (22) is operated with a accumulator or a battery.

23. Medical treatment apparatus according to claim 20 and claim 2 or 15,
**characterized in that**,
with the aid of the foot switch (25) the drive speed and direction of rotation of the drive motor (23) can be altered.

## Revendications

1. Dispositif de traitement médical (20), comportant un appareil de commande (21),
avec une conduite d'alimentation (22) pouvant être raccordée à l'appareil de commande (21), par l'intermédiaire de laquelle l'appareil de commande (21) commande le fonctionnement d'un outil de traitement (39), et
avec des moyens de sélection (1, 2) pour choisir entre une pluralité de premiers programmes d'exploitation formellement préétablis, ne pouvant pas être modifiés par un utilisateur, d'une part, et une pluralité de seconds programmes d'exploitation pouvant être modifiés à volonté par l'utilisateur d'autre part,
moyennant quoi l'appareil de commande, commande le fonctionnement de l'outil de traitement (39) en fonction des informations de paramètres de fonctionnement mises à disposition par un premier ou un deuxième programme d'exploitation sélectionné.

2. Dispositif de traitement médical selon la revendication 1,
**caractérisé en ce que**
un moteur d'entraînement (23) est raccordé à la conduite d'alimentation (22) avec lequel peut être couplé un instrument de traitement (38, 40) moyennant quoi l'outil de traitement (39) peut à nouveau être couplé à l'instrument de traitement (38, 40).

3. Dispositif de traitement médical selon l'une des revendications précédentes,
**caractérisé en ce que**
à l'aide des moyens de sélection (1, 2, 3) un mode d'ajustement peut de plus être sélectionné, dans lequel des paramètres d'exploitation déterminés peuvent être ajustés par l'utilisateur.

4. Dispositif de traitement médical selon la revendication 2,
**caractérisé en ce que**,
une banque de données (41,42) est mémorisée dans l'appareil de commande (21) qui comprend des informations sur les instruments de traitement disponibles (38,40) et/ou les outils de traitement disponibles (39).

5. Dispositif de traitement médical selon la revendication 4,
**caractérisé en ce que**,
pour chaque instrument de traitement disponible (38,40) la banque de données (41) comprend des informations sur la transmission du nombre de tours, le degré d'efficacité, le couple de démarrage maximum, le nombre de tours d'entraînement maximum et/ou la plage de nombre de tours de fonctionnement prévue.

6. Dispositif de traitement médical selon la revendication 3 et la revendication 4 ou 5,
**caractérisé par**
des moyens de réglage (6-9) pour ajuster un instrument de traitement souhaité (38,40) et/ou un outil de traitement souhaité (39) dans le mode de réglage sur la base des informations introduites dans la banque de données (41,42).

7. Dispositif de traitement médical selon l'une des revendications précédentes,
**caractérisé par**
un dispositif de pompe de réfrigérant (31,61) pour amener un réfrigérant à l'outil de traitement (39).

8. Dispositif de traitement médical selon les revendications 6 et 7,
**caractérisé en ce que**,
la banque de données (41-43) comprend également des informations sur les capacités de pompe possibles ou les quantités de réfrigérant possibles du dispositif de pompe de réfrigérant (31,61) et **en ce qu'**à l'aide des moyens de réglage (6-9,13) sur la base des informations introduites dans la banque de données (41-43), on peut ajuster une capacité de pompe souhaitée ou une quantité de réfrigérant du dispositif de pompe de réfrigérant (31,61).

9. Dispositif de traitement médical selon la revendication 6 ou 8,
**caractérisé par**
des moyens de mémorisation (5) pour mettre en mémoire après le réglage des paramètres de fonctionnement déterminés à l'aide des moyens de réglage (6-9,13) les paramètres de fonctionnement réglés en tant que second programme de fonctionnement.

10. Dispositif de traitement médical selon la revendication 2,
**caractérisé en ce que**,
les informations de paramètres de fonctionnement correspondant à un premier ou à un second programme de fonctionnement comprennent le type d'un instrument de traitement déterminé (38,40), la transmission du nombre de tours, le degré d'efficacité, le couple de démarrage maximum, le nombre de tours d'entraînement maximum et/ou la plage du nombre de tours de fonctionnement prévue de l'instrument de traitement déterminé (38,40) ainsi que le type d'un outil de traitement déterminé (39).

11. Dispositif de traitement médical selon l'une des revendications précédentes,
**caractérisé par**
une unité d'affichage (36) pour afficher selon la sélection d'un premier ou d'un second programme de fonctionnement les informations de paramètres de fonctionnement correspondant au programme de fonctionnement choisi.

12. Dispositif de traitement médical selon la revendication 11 et l'une des revendications 6, 8 ou 9,
**caractérisé en ce que**,
l'unité d'affichage (36) affiche également l'instrument de traitement (38,40) ou l'outil de traitement réglés à l'aide des moyens de traitement (39).

13. Dispositif de traitement médical selon la revendication 11 ou 12,
**caractérisé en ce que**
l'unité d'affichage (36) affiche également les messages d'erreurs et/ou les informations auxiliaires.

14. Dispositif de traitement médical selon la revendication 2,
**caractérisé en ce que**,
le moteur d'entraînement (23) est un moteur sans collecteur avec une plage de nombre de tours d'entraînement de 200 tr/min à 40 000 tr/min.

15. Dispositif de traitement médical selon la revendication 2 ou 14,
**caractérisé en ce que**,
sur l'appareil de commande (21) est raccordé un second moteur d'entraînement (21).

16. Dispositif de traitement médical selon la revendication 7 et 15,
**caractérisé par**
un second dispositif de pompe de réfrigérant (31,61) pour amener un réfrigérant à un outil de traitement (39) entraîné par le second moteur d'entraînement (23).

17. Dispositif de traitement médical selon l'une des revendications précédentes,
**caractérisé par**
un dispositif d'entraînement pneumatique (33) pour entraîner pneumatiquement un outil de traitement (34) raccordable à celui-ci.

18. Dispositif de traitement médical selon la revendication 17,
**caractérisé en ce que**,
la pression de l'air d'entraînement fourni par le dispositif d'entraînement pneumatique (33) est réglable.

19. Dispositif de traitement médical selon l'une des revendications précédentes,
**caractérisé par**
une interface pour la connexion à une imprimante pour imprimer des informations de fonctionnement déterminées du dispositif de traitement médical sur l'imprimante.

20. Dispositif de traitement médical selon l'une des revendications précédentes,
**caractérisé par**
un commutateur à pédale sans câble (25) qui transmet les signaux de commande à l'appareil de commande (21) pour le pilotage de l'appareil de commande.

21. Dispositif de traitement médical selon la revendication 20,
**caractérisé en ce qu'**à l'aide du commutateur à pédale (25) on peut sélectionner un programme de fonctionnement souhaité à partir des premier et second programmes de fonctionnement.

22. Dispositif de traitement médical selon la revendication 20 ou 21,
**caractérisé en ce que**,
le commutateur à pédale (25) est entraîné par un accumulateur ou une batterie.

23. Dispositif de traitement médical selon la revendication 20 et la revendication 2 ou 15,
**caractérisé en ce que**,
le nombre de tours d'entraînement et le sens de rotation du moteur d'entraînement (23) peut être modifié à l'aide du commutateur à pédale (25).
